# EUROPÄISCHE PATENTSCHRIFT

(11) **EP 2 658 444 B1**
(45) Veröffentlichungstag und Bekanntmachung des Hinweises auf die Patenterteilung: **05.11.2014**
(21) Anmeldenummer: 11794195.5
(22) Anmeldetag: 14.12.2011
(51) Int. Cl.: A61B 5/145, A61B 5/1486, A61N 1/05

(54) **VERFAHREN ZUR BEREITSTELLUNG EINES EFFIZIENTEN BIOSENSORS, SOWIE ENTSPRECHENDE BIOSENSOR, SUBSTRAT UND INSERTIONSKIT**
METHOD FOR PROVIDING AN EFFICIENT BIOSENSOR, AS WELL AS CORRESPONDING BIOSENSOR, SUBSTRATE AND INSERTION KIT
PROCÉDÉ DE MISE AU POINT D'UN BIOCAPTEUR EFFICACE, AINSI QUE BIOCAPTEUR, SUBSTRAT ET KIT D'INTRODUCTION CORRESPONDANTS

(30) Priorität: 30.12.2010 EP 10197413
(43) Veröffentlichungstag der Anmeldung: 06.11.2013
(73) Patentinhaber: Roche Diagnostics GmbH, 68305 Mannheim (DE); F.Hoffmann-La Roche AG, 4070 Basel (CH)
(72) Erfinder: FREY, Stephan-Michael, 64347 Griesheim (DE); KUBE, Oliver, 67549 Worms (DE)
(74) Vertreter: Stößel, Matthias
(86) Internationale Anmeldenummer: PCT/EP2011/072732
(87) Internationale Veröffentlichungsnummer: WO 2012/089505

(56) Entgegenhaltungen:
- EP-A1- 1 870 127
- EP-A1- 1 909 098
- WO-A1-2010/114998
- WO-A2-2006/116765
- US-A1- 2006 024 774

## Beschreibung

### Gebiet der Erfindung

Die Erfindung liegt auf dem Gebiet der Biosensoren, im Speziellen der Biosensoren für kontinuierliche Messung von Körperflüssigkeitsparametern im Körper eines Benutzers. Die Erfindung betrifft einen Biosensor zum Insertieren in ein subcutanes Gewebe eines Benutzers. Die Erfindung betrifft weiterhin einen Substratzuschnitt zur Herstellung eines Biosensors und ein Insertionskit zum Insertieren eines Biosensors in ein subcutanes Gewebe eines Benutzers sowie ein Verfahren zur Herstellung eines Biosensors.

### Stand der Technik

Aus dem Stand der Technik sind, neben so genannten Punktmessungen, die lediglich einmalig oder wenige Male durchgeführt werden, unter anderem auch Langzeitüberwachungen eines oder mehrerer physiologischer Parameter bekannt. Die Erfindung wird im Folgenden im Wesentlichen beschrieben unter Bezugnahme auf physiologische Parameter in Form von Analytkonzentrationen eines oder mehrerer Analyte in einer Körperflüssigkeit des Benutzers, beispielsweise eines menschlichen oder tierischen Patienten, unabhängig davon, ob tatsächlich eine Krankheit vorliegt oder ob lediglich eine Überwachung gesunder Benutzer stattfinden soll. Ohne Beschränkung weiterer möglicher Anwendungen wird die Erfindung im Folgenden unter Bezugnahme auf eine Blutglucoseüberwachung beschrieben. Grundsätzlich ist die Erfindung jedoch auch auf andere Arten von Analyten und/oder die Überwachung anderer Arten von physiologischen Parametern übertragbar.

In jüngerer Zeit etabliert sich mehr und mehr eine kontinuierliche Glucosemessung im Interstitium des Benutzers, welche auch als Continuous Monitoring, CM, bezeichnet wird. Dieses Verfahren eignet sich zum Management, zur Überwachung und zur Steuerung beispielsweise eines Diabetes-Status. Mittlerweile sind dabei aus dem Stand der Technik direkt implantierte elektrochemische Sensoren bekannt, welche häufig auch als nadelartige Sensoren (needle type sensors, NTS) bezeichnet werden. Dabei wird der aktive Sensorbereich direkt an einen Messort gebracht, welcher in der Regel im interstitiellen Gewebe angeordnet ist und beispielsweise unter Verwendung eines Enzyms (beispielsweise Glucoseoxidase, GOD oder Glucosedehydrogenase) Glucose in elektrische Ladung umsetzt, die im Verhältnis zur Glucose-Konzentration steht und als Messgröße verwendet werden kann. Beispiele derartiger transkutaner Messsysteme sind in US 6,360,888 B1 oder in US 2008/0242962 A1 beschrieben. Continuous Monitoring-Systeme erfassen in der Regel in regelmäßigen oder unregelmäßigen Zeitabständen Messwerte, beispielsweise Glucosemesswerte. Beispielsweise können bei implantierten Sensoren im Abstand von 5 min oder weniger Glucosemesswerte erfasst werden.

Zur Vermessung von Körperflüssigkeitsparametern im Körper eines Benutzers sind verschiedene Sensoren im Stand der Technik bekannt, die auf Basis von elektrochemischen Biosensoren verschiedene Moleküle kontinuierlich umsetzen können und Messergebnisse für diese Parameter bestimmen können. So ist beispielsweise aus der US 6,103,033 ein elektrochemischer Biosensor bekannt, der zur kontinuierlichen Vermessung von Glucose in den Patienten eingebracht werden kann. Dieser Biosensor besitzt auf einer planaren Oberfläche eines flexiblen Substrats mehrere Elektroden, die über Leiterbahnen mit Kontaktierungselementen verbunden sind. Hiermit ist die Vermessung eines an den Elektroden umgesetzten Parameters möglich, der an den Elektroden elektrische Signale erzeugt. Diese werden durch die Kontaktierung der Kontaktierungselemente mit einem Messgerät in einen Parameterwert umgewandelt, der dem Benutzer zur Verfügung gestellt wird.

Auch die US 2008/0135408 beschäftigt sich mit einem Biosensor mit einer einfachen Anordnung von Arbeitselektrode, Gegenelektrode und Referenzelektrode auf einem planaren Substrat, wobei wiederum die Elektroden mit Kontaktierungselementen verbunden sind, die wiederum an ein Messgerät angeschlossen werden können. Darüber hinaus gibt es Biosensoren, die nicht nur eine Arbeitselektrode aufweisen, sondern über eine Verteilung einer Arbeitselektrode über verschiedene Orte auf der Oberfläche des Sensors verfügen, und darüber hinaus mehrere solcher Arbeitselektroden auf der Vorder- und Rückseite eines Biosensors aufweisen können.

Auch die WO 2010/014959 beschreibt Möglichkeiten, mehrere Elektrodenpaare aus Arbeits- und Gegenelektrode auf einer Substratoberfläche anbringen zu können.

Des Weiteren beschreiben die EP 1 870 127 A1, WO 2006/116765 A2 sowie die WO 2010/114998 A1 Multielektroden-Arrays, die dazu dienen in den Körper eingebracht zu werden, um eine Stimulation von Gewebe vorzunehmen oder Schmerzen zu therapieren.

Auch wenn im Stand der Technik bereits Möglichkeiten aufgezeigt wurden, wie kontinuierlich messende Biosensoren, die in den Körper des Patienten eingebracht werden müssen, möglichst effizient mit verschieden geometrisch geformten und angebrachten Elektroden zu versehen, so zeigt der Stand der Technik keine angepassten Möglichkeiten auf, Biosensoren mit mehr als einer Elektrode effizient und kostengünstig herzustellen. Da mit üblichen Druckverfahren zum Aufbringen von elektrischen Bauelementen, wie beispielsweise Druckverfahren wie beispielsweise dem Siebdruck, jeweils nur eine Seite einer Oberfläche des Substrats bedruckt werden kann, weisen die Systeme aus dem Stand der Technik mit mehr als einer bedruckten Oberfläche den Nachteil auf, dass hierfür mehrere Prozessschritte auf beiden Substratoberflächen wiederholt werden müssen. Dies führt zu einem sehr aufwändigen und kostenintensiven Herstellprozess für Biosensoren mit mehr als einer Substratoberfläche, die mit Elektroden, Kontaktierungselementen und Leiterbahnen versehen sind. Zudem werden im Stand der Technik ausschließlich Systeme beschrieben, die nur einen Träger besitzen, auf dem die elektrischen Bauelemente aufgebracht werden. Dies begrenzt die Möglichkeit, über einer vorgegebenen Länge des zu insertierenden Teils des NTS mehr elektrische Bauelemente auf das Substrat aufzubringen als diese vorgegebene Länge zulässt.

### Aufgabe der Erfindung

Es ist daher eine Aufgabe der vorliegenden Erfindung, Vorrichtungen und Verfahren anzugeben, welche die Nachteile bekannter Vorrichtungen und Verfahren zumindest weitgehend vermeiden. Insbesondere soll ein Verfahren zur Herstellung eines Biosensors angegeben werden, welches auf einfache Weise das Aufbringen einer Vielzahl elektrischer Bauelemente, wie beispielsweise Kontaktierungselemente, Leiterbahnen und Elektroden, auf eine Oberfläche eines Sensorsubstrats ermöglichen kann. Im Besonderen soll ein Verfahren angegeben werden, welches einen Schritt aufweist, der es ermöglicht, die Oberfläche, auf die die verschiedenen Elemente aufgebracht werden sollen, in mehrere miteinander verbundene Außenoberflächen des Biosensors zu unterteilen.

### Offenbarung der Erfindung

Diese Aufgabe wird gelöst durch die Erfindung mit den Merkmalen der unabhängigen Patentansprüche. Vorteilhafte Weiterbildungen der Erfindung sind in den abhängigen Patentansprüchen dargestellt.

In einem ersten Aspekt der vorliegenden Erfindung wird ein Biosensors zum Insertieren in ein subcutanes Gewebe eines Benutzers vorgeschlagen, wobei der Biosensor mindestens ein flexibles Substrat und mindestens eine Elektrode auf mindestens einer Oberfläche des Substrats und mindestens ein Kontaktierungselement aufweist, wobei das Kontaktierungselement mit der Elektrode verbunden ist, wobei das Substrat mindestens eine Knickstelle aufweist, an der das Substrat mindestens zu einem Teil geknickt ist, so dass die Oberfläche in zumindest zwei miteinander verbundene Außenoberflächen unterteilt ist.

Der Biosensor ist, wie bereits oben erwähnt, zum Insertieren in ein subcutanes Gewebe eines Benutzers ausgestaltet, wobei beispielsweise biokompatible Materialien verwendet werden können, zumindest auf einer Außenseite des Biosensors, welcher im insertierten Zustand mit dem Körpergewebe in Kontakt kommt.

Das Substrat kann, vor allem vor dem Knickvorgang, insbesondere planar ausgestaltet sein. Nach dem Knickvorgang kann das Substrat beispielsweise wiederum eine planare Form aufweisen. Alternativ kann das Substrat nach dem Knickvorgang aber auch in eine oder zwei weitere Dimensionen geknickt vorliegen. So kann der Sensor bei Benutzung entweder eine im Wesentlichen zweidimensionale Form aufweisen oder sogar eine dreidimensionale Form besitzen. Unter einem planaren Substrat wird im Rahmen der vorliegenden Erfindung ein Substrat verstanden, welches mindestens eine im Wesentlichen ebene Oberfläche aufweist, vorzugsweise mindestens zwei einander gegenüberliegende und vorzugsweise im Wesentlichen parallele im Wesentlichen ebene Oberflächen. Die bevorzugte planare Ausgestaltung des Substrats soll sich dabei auf den Herstellungsvorgang beziehen, wobei der eigentliche Biosensor im späteren Einsatz auch ganz oder teilweise gekrümmt ausgestaltet sein kann, beispielsweise unter Einwirkung von Kräften durch das Körpergewebe und/oder unter Einwirkung von Kräften durch eine oder mehrere mechanische Vorrichtungen, beispielsweise ein Bodymount, welches den Biosensor auf einer Hautoberfläche des Benutzers hält und welches gegebenenfalls einen Teil des Biosensors und/oder des Substrats biegen kann, beispielsweise zum Zweck einer Kontaktierung. In einem Ruhezustand, ohne Einwirkung äußerer Kräfte, sind das Substrat und/oder der Biosensor jedoch im Wesentlichen eben ausgestaltet. Unter im Wesentlichen eben wird dabei ein ebenes Substrat verstanden, wobei jedoch auch leichte Abweichungen von einer ebenen Ausgestaltung tolerierbar sind. Beispielsweise können Oberflächenkrümmungen mit einem Krümmungsradius von mehr als 10 mm, vorzugsweise von mehr als 20 mm und insbesondere von mehr als 30 mm oder mehr als 50 mm als eben angesehen werden. Weiterhin kann die im Wesentlichen ebene Oberfläche vorzugsweise glatt ausgestaltet sein, wobei jedoch auch leichte Rauigkeiten tolerierbar sind, beispielsweise eine quadratische Rauigkeit (rms) von weniger als 1 mm, insbesondere von weniger als 0,5 mm. Unter im Wesentlichen parallel wird eine vollständige Parallelität verstanden, wobei jedoch auch Abweichungen von nicht mehr als ±20°, insbesondere von nicht mehr als ±10° und besonders bevorzugt von nicht mehr als ±5° im Rahmen des genannten Begriffs tolerierbar sein können. Besonders bevorzugt weist das vorzugsweise planare Substrat eine Dicke auf, welche erheblich geringer ist als die laterale Ausdehnung des vorzugsweise planaren Substrats, beispielsweise eine Dicke, welche eine Länge und/oder eine Breite des planaren Substrats um mindestens einen Faktor 10 unterschreitet, vorzugsweise um mindestens einen Faktor 100. Beispielsweise kann das vorzugsweise planare Substrat eine Dicke von nicht mehr als 4 mm aufweisen, insbesondere eine Dicke von nicht mehr als 3 mm und besonders bevorzugt von nicht mehr als 2 mm oder sogar nicht mehr als 1 mm. Vorzugsweise ist das vorzugsweise planare Substrat somit ein dünnes Substrat mit zwei einander gegenüberliegenden, im Wesentlichen parallelen und im Wesentlichen ebenen Oberflächen.

Unter einem flexiblen Substrat wird im Rahmen der vorliegenden Erfindung ein Substrat verstanden, welches unter üblichen, im Betrieb und insbesondere im insertierten Zustand auftretenden Kräften verformt wird, beispielsweise bei Kräften von 1 bis 10 Newton, beispielsweise Biegekräften. Die Verformung kann reversibel oder auch irreversibel sein. Die Verformung kann plastischer oder auch elastischer Art sein. Die Verformung kann durch geeignete Wahl eines Substratmaterials und/oder durch geeignete Wahl einer Geometrie des Substrats bewirkt werden, beispielsweise durch ein dünnes, längliches Substrat.

Das Substrat kann beispielsweise aus mindestens einem Substratmaterial hergestellt sein. Dieses Substratmaterial kann beispielsweise ausgewählt sein aus einem Papiermaterial, einem Kunststoffmaterial, einem Keramikmaterial oder einer Kombination der genannten und/oder anderer Materialien. Auch mehrschichtige Substratmaterialien können eingesetzt werden, beispielsweise Laminate. Das Substrat des Biosensors kann beispielsweise mit einem oder mehreren Funktionselementen versehen sein, welche beispielsweise auf einer oder mehreren Oberflächen aufgebracht sein können. Beispielsweise kann es sich hierbei, wie unten noch näher ausgeführt wird, um eine oder mehrere Elektroden und/oder ein oder mehrere Kontaktierungselemente handeln. Auch andere Funktionselemente können vorgesehen sein. Diese Funktionselemente können mittels einer oder mehrerer grundsätzlich bekannter Techniken aufgebracht werden, beispielsweise Drucktechniken und/oder anderen Techniken, insbesondere Beschichtungstechniken, beispielsweise physikalischer und/oder chemischer Dampfphasenabscheidung.

Der Biosensor besitzt auf mindestens einer Oberfläche seines Substrates jeweils mindestens eine Elektrode und jeweils mindestens ein Kontaktierungselement, wobei das Kontaktierungselement mit der Elektrode über beispielsweise eine Leiterbahn verbunden ist. Die mindestens eine Elektrode kann insbesondere mindestens eine Arbeitselektrode und/oder mindestens eine Referenzelektrode und/oder mindestens eine Gegenelektrode umfassen. Die Elektrode kann beispielsweise mindestens eine elektrisch leitende Elektrodenfläche umfassen. Weiterhin kann die Elektrode zusätzliche Elemente umfassen, beispielsweise mindestens eine Beschichtung, beispielsweise eine Enzymbeschichtung und/oder mindestens ein Redox-System. Derartige Elektroden sind aus dem Stand der Technik grundsätzlich bekannt. Auch andere Ausgestaltungen sind jedoch grundsätzlich möglich.

Bevorzugt weist der Biosensor mindestens eine Arbeitselektrode auf mindestens einer der Oberflächen auf.

Das Substrat weist weiterhin die mindestens eine Knickstelle auf, an der das Substrat mindestens zu einem Teil geknickt wird. Die Knickstelle kann grundsätzlich eine beliebige geometrische Gestalt aufweisen. Beispielsweise kann die Knickstelle mindestens eine Knicklinie umfassen, entlang derer das Substrat beispielsweise linear geknickt wird. Auch komplexere Geometrien sind jedoch möglich. Das Knicken kann grundsätzlich mit einem beliebigen Radius erfolgen, vorzugsweise jedoch mit einem Radius von nicht mehr als 5 mm, insbesondere nicht mehr als 3 mm und besonders bevorzugt von nicht mehr als 1 mm. Außerhalb der Knickstelle können die Außenoberflächen beispielsweise im Wesentlichen eben ausgestaltet sein.

Bei dem Knickvorgang wird mindestens ein Teil des Substrats gegenüber dem anderen Teil des Substrats abgeknickt, so dass die beiden Teile nicht mehr miteinander fluchtend angeordnet sind. Hierdurch wird die Oberfläche, auf der beispielsweise sämtliche Elemente zur Messung mit dem Biosensor angebracht sein können, in mindestens zwei Teile unterteilt. Da bei diesem Knickvorgang die beiden Teile des Substrates bzw. Biosensors vorzugsweise aufeinander zu bewegt werden, entstehen vorzugsweise mindestens zwei Innenoberflächen, die sich beim Knickvorgang auf einander zu bewegen und zueinander weisen. Die den beiden optionalen Innenoberflächen jeweils gegenüberliegenden Oberflächen auf dem gleichen Substratteil werden als Außenoberflächen des Substrates bezeichnet. Diese mindestens zwei Außenoberflächen weisen voneinander weg.

Unter einem Knicken ist im Rahmen der vorliegenden Erfindung ein Vorgang zu verstehen, bei welchem eine Oberfläche eines Körpers, beispielsweise des Substrats, durch Einwirkung mechanischer Kräfte zumindest weitgehend irreversibel verformt wird. Insbesondere kann bei dem Knicken eine Knickstelle entstehen, an der eine Oberflächenkrümmung des Substrats eine sprunghafte Änderung durchläuft. Zumindest weitgehend irreversibel bedeutet in diesem Zusammenhang, dass sich das Substrat nach dem Knickvorgang ohne Einwirkung von Kräften bevorzugt nicht von sich aus wieder in die ursprüngliche Form vor dem Knicken zurück bewegt. Die Knickstelle bewirkt bevorzugt eine stabil bleibende Abwinklung der beiden abgeknickten Oberflächen bzw. Außenoberflächen. Das Knicken ist ein aktiver, bewusster Vorgang, der in seiner Dimensionierung und Richtung gezielt gewählt werden kann. Durch den Knickvorgang werden die beiden abgeknickten Außenoberflächen vorzugsweise in einem bestimmten Winkel zu einander ausgerichtet. Der Winkel kann frei gewählt werden, wie später noch näher erläutert wird. Die resultierende Fixierung der Außenoberflächen kann beispielsweise dadurch bewirkt werden, dass das Substrat zumindest zu einem Teil aus einem nicht elastischen Material besteht. Alternativ oder zusätzlich können die beiden abgeknickten Teile nach dem Knicken zumindest an der Knickstelle zueinander fixiert werden. Das Fixieren kann beispielsweise ein Verkleben oder Anheften der beiden abgeknickten Teile, bevorzugt an den Innenoberflächen, sein. Bevorzugt ist, dass das Substrat zumindest an der Knickstelle nach dem Knickvorgang nicht in oder entgegen der Knickrichtung weiter verbiegbar ist. Das Substrat kann ansonsten elastisch und verbiegbar ausgestaltet sein, sodass sich der restliche Biosensor bei Benutzung dem Körper, in den er insertiert wird, anpassen kann, ohne dass der Biosensor seine geknickte Grundstruktur verliert. Das hat zur Folge, dass der Biosensor sich nach Einwirkung von äußeren Kräften, die einen Teil des Substrates des Biosensors verbiegen, bevorzugt seine ursprüngliche Form nach dem Knickvorgang wieder annimmt. Die Knickstelle kann innerhalb des Substrates eine Unstetigkeit darstellen, da das Substrat bevorzugt an dieser Stelle eine abrupte Richtungsänderung seiner Oberfläche vornimmt. Dies steht im Gegensatz zu der stetigen Rich-tungsänderung bei einem Biegevorgang beispielsweise des restlichen Substrates beispielsweise beim Insertieren und Benutzen. Der geknickte Biosensor, folglich nach dem Knickvorgang der Oberfläche, wird im Folgenden auch als gefalteter Biosensor bezeichnet.

Bei dem Knicken wird weiterhin bevorzugt mindestens eine elektrisch leitende Komponente mit abgeknickt und/oder gefaltet. Die elektrisch leitende Komponente kann beispielsweise mindestens ein Teil ausgewählt aus der Gruppe bestehend aus einem Kontaktierungselement, einer Elektrode, einer Leiterbahn und weiterer Funktionselemente oder mindestens zweier davon sein. Struktur und Aufbau dieser elektrisch leitenden Komponenten wird später noch erläutert.

Durch das Knicken des Substrates des Biosensors kann erreicht werden, dass mehr als nur eine Oberfläche des zu insertierenden Biosensors mit Elektroden und Kontaktierungselementen belegt werden kann, ohne dabei mehr als eine Oberfläche vor dem Knicken des Biosensorsubstrates mit aktiven Elementen zu belegen. Beispielsweise kann mindestens eine einseitige Beschichtung des Substrats vor dem Knickvorgang erfolgen, wobei nach dem Knickvorgang dennoch ein beidseitig beschichteter Biosensor entstehen kann. Es ist jedoch weiterhin denkbar, auch die Innenseiten des Biosensors mit elektrischen Bauelementen, wie Elektroden und Kontaktierungselementen zu belegen.

Bevorzugterweise wird der Biosensor bei dem Knickvorgang um einen Winkel von im Wesentlichen 180° geknickt, also beispielsweise um einen Winkel von 180° ± 30°, vorzugsweise 180° ± 20°, insbesondere 180° ± 10° oder sogar 180° ± 5°. Dies hat zur Folge, dass die beiden Innenflächen beispielsweise aufeinander bzw. nahezu aufeinander liegen können.

Die Erfindung kann folglich derart ausgestaltet sein, dass der Biosensor oder dessen Substrat derart ausgestaltet wird, beispielsweise derart verlängert wird, dass mindestens zwei Sensorzungen entstehen, beispielsweise Sensorzungen in Form der Außenoberflächen, die bevorzugt jeweils nur einseitig beschichtet, beispielsweise bedruckt, werden. Dadurch können auf dem Biosensor beispielsweise doppelt so viele Elektrodenpaare, beispielsweise bestehend aus Arbeitselektrode und Gegenelektrode, auf der gleichen Fläche des Biosensors untergebracht werden wie bei herkömmlichen Biosensoren. Der Biosensor kann nach der Beschichtung gefaltet bzw. geknickt werden und kann anschließend mit einem Stecker verklebt werden, der die Kontaktierung mit einer Messeinheit gewährleistet kann.

Das Substrat kann, vor allem vor dem Knickvorgang, insbesondere planar ausgestaltet sein. Nach dem Knickvorgang kann das Substrat beispielsweise wiederum eine planare Form aufweisen, wie oben durch das Knicken um 180° beschrieben. Alternativ kann das Substrat nach dem Knickvorgang, insbesondere einem ersten Knickvorgang, beispielsweise auch nicht-eben ausgestaltet sein. Beispielsweise kann dies durch einen oder mehrere Knickvorgänge mit einem von 180° abweichenden Knickwinkel geschehen. Beispielsweise kann das Substrat nach dem Knickvorgang in eine oder zwei weitere Richtungen geknickt vorliegen. So kann der Sensor bei Benutzung entweder eine im Wesentlichen zweidimensionale Form aufweisen oder sogar eine dreidimensionale Form besitzen. Eine dreidimensionale Form kann beispielsweise dadurch erreicht werden, dass das Substrat mehr als eine Knickstelle aufweist, um die das Substrat zum einsatzbereiten Sensor geknickt wird. Diese weitere bzw. weiteren Knickstellen können sich parallel zu der ersten Knickstelle auf bzw. an dem Substrat befinden oder in einem geneigten Winkel. Dieser Winkel zwischen der ersten und den weiteren Knickstellen kann beispielsweise zwischen 1° und 90° liegen, beispielsweise je nachdem welche dreidimensionale Struktur der Sensor haben soll. Das Substrat kann beispielsweise an der ersten Knickstelle einen ersten Knickvorgang erfahren und an einer zweiten, von der ersten Kickstelle entfernten Knickstelle, einen zweiten Knickvorgang erfahren. Alternativ kann das Substrat im zweiten Knickvorgang auch quer zum ersten Knickvorgang geknickt werden. Die erste Knickstelle kann dabei selbst mit geknickt werden oder die zweite Knickstelle befindet sich in einem anderen Bereich.

Es können weitere Knickvorgänge parallel, schräg oder quer zu einem der ersten beiden Knickvorgänge gemacht werden, sodass ein Sensor mit einer mehrfach gefalteten dreidimensionalen Struktur entsteht.

Bevorzugt wird ein zweiter Knickvorgang an einer zweiten Knickstelle durchgeführt, die parallel zur ersten Knickstelle angeordnet ist. Die beiden Knickvorgänge können zeitgleich erfolgen oder nacheinander durchgeführt werden. Bevorzugt wird das Substrat bei den beiden Knickvorgängen jeweils um etwa 90° bis 150°, besonders bevorzugt um 110° bis 130° geknickt. Werden die beiden Knickvorgänge in die gleiche Richtung vorgenommen, so liegt in einer bevorzugten Ausführungsform ein dreieckig geformter Biosensor vor. Der Biosensor mit zwei parallelen Knicklinien weist nach dem Knickvorgang drei Außenoberflächen auf, die jeweils mit Funktionselementen, wie Elektroden oder Kontaktierungselementen versehen sein können. Es kann beispielsweise nur eine der drei Außenoberflächen mit Elektroden und Kontaktierungselement versehen. Alternativ können zwei oder auch drei der Außenoberflächen mit Elektroden und Kontaktierungselementen versehen sein. In einer alternativen Ausführungsform ist nur auf einer der drei Außenoberflächen ein Kontaktierungselement angebracht, das mit mehreren Arbeitselektroden auf zwei oder drei Außenoberflächen über Leiterbahnen verbunden ist. Zusätzlich kann eine, zwei oder jede Außenoberfläche eine Gegenelektrode und/oder eine Referenzelektrode aufweisen.

Zusätzlich können auch noch weitere parallele oder schräge Knickstellen zu der ersten oder zweiten Knickstelle eingebracht werden. Die beiden bzw. alle Knickvorgänge können entweder nacheinander oder zeitlich parallel zueinander erfolgen.

Es können sich auf allen nach den diversen Knickvorgängen entstandenen Außenoberflächen ein oder mehrere Funktionselemente, wie Arbeitselektrode, Gegenelektrode, Referenzelektrode oder Kontaktierungselement befinden. So kann beispielsweise auf einer Außenoberfläche sowohl ein Kontaktierungselement als auch eine Arbeitselektrode, verbunden durch eine Leiterbahn, angeordnet sein. Weiterhin kann auf dieser Außenoberfläche eine Gegenelektrode und/oder eine Referenzelektrode bzw. eine Kombination aus Gegen-, und Referenzelektrode angeordnet sein. Es sind jedoch auch alle weiteren Kombinationen von Funktionselementen auf den verschiedenen Außenoberflächen und Innenflächen des Biosensors vorstellbar. So können Elektroden auf einer Außenoberfläche durch Leiterbahnen mit mindestens einem Kontaktierungselement auf einer anderen Außenoberfläche oder eine Innenfläche des Biosensors verbunden sein.

Der Biosensor kann insbesondere derart ausgestaltet sein, dass auf mindestens zwei der Außenoberflächen jeweils mindestens eine Elektrode angeordnet ist.

Vorzugsweise kann zwischen dem mindestens einen Kontaktierungselement und der mindestens einen Elektrode mindestens eine Leiterbahn angeordnet sein. Insbesondere wenn mehrere Kontaktierungselemente und mehrere Elektroden vorgesehen sind, so können auch mehrere Leiterbahnen vorgesehen sein.

In einer bevorzugten Ausführungsform ist der Biosensor derart ausgestaltet, dass die Außenoberflächen mindestens in zwei zueinander im Wesentlichen parallel angeordneten Ebenen liegen. Im Wesentlichen bedeutet in diesem Zusammenhang im Rahmen der vorliegenden Erfindung, dass die beiden Außenoberflächen so zueinander angeordnet sind, dass außer im Bereich der Knickstelle die beiden Außenoberflächen parallel zueinander verlaufen. Es kann eine Abweichung von nicht mehr als 20°, bevorzugt von nicht mehr als 10°, besonders bevorzugt von nicht mehr als 5° von dieser 180°-Anordnung der beiden Außenoberflächen vorliegen, was noch unter den Begriff im Wesentlichen parallel angeordnet subsumierbar sein soll.

Der Biosensor kann insbesondere derart ausgestaltet sein, dass durch die Knickstelle das Substrat in mindestens zwei miteinander verbundene Substratteile unterteilt wird, wobei die Substratteile mindestens zu einem Teil miteinander verbunden sind, insbesondere verklebt. Um eine Fixierung der beiden Substratteile und damit auch der beiden Außenoberflächen zueinander zu ermöglichen, können insbesondere die beiden Innenflächen miteinander verklebt werden. Das Verkleben kann beispielsweise durch kurzzeitiges Erhitzen des Substrats zur Erweichung des Substratmaterials und anschließendes Aneinanderdrücken geschehen. Alternativ können ein oder mehrere Kleber benutzt werden, um die Innenflächen miteinander zu verbinden.

Nach dem Knickvorgang können sowohl auf einer als auch auf beiden Außenoberflächen ein oder mehrere Elemente wie beispielsweise Elektroden, Kontaktierungselemente sowie Leiterbahnen angebracht sein. Sind auf beiden Außenoberflächen Elemente angebracht, so können ein oder mehrere Elemente auf der einen Außenoberfläche auch mit einem oder mehreren Elementen auf der anderen Außenoberfläche verbunden sein, beispielsweise mittels eines oder mehrerer Verbindungselemente, welche sich über die Knickstelle hinweg erstrecken können. Beispielsweise können dies ein oder mehrere Leiterbahnen sein. So ist beispielsweise vorstellbar, dass auf einer ersten Außenoberfläche mindestens eine Elektrode aufgebracht wurde und auf der zweiten Außenoberfläche mindestens ein Kontaktierungselement aufgebracht wurde, die durch eine Leiterbahn, welche sich über die Knickstelle hinweg zwischen der mindestens einen Elektrode und dem mindestens einen Kontaktierungselement erstreckt, verbunden sind.

Alternativ oder zusätzlich zu einer stoffschlüssigen Verbindung der beiden Substratteile können eine oder mehrere andere Verbindungstechniken eingesetzt werden, beispielsweise kraftschlüssige und/oder formschlüssige Verbindungen. So besteht eine Möglichkeit, die beiden Substratteile des Biosensors nach dem Knicken gegeneinander zu fixieren, in einer Bereitstellung eines oder mehrerer Stege zwischen den Außenoberflächen. So kann beispielsweise durch ein weiteres Knicken des Substrates an einem oder beiden Enden des geknickten Biosensors eine Fixierung der zwei Substratteile zueinander erreicht werden. Zusätzlich oder alternativ hierzu können Stege an einem Teil des Substrates angebracht sein, die nach dem ersten Knickvorgang um das geknickte Substrat geknickt werden, um so eine Fixierung der beiden abgeknickten Substratteile, beispielsweise durch einen Formschluss und/oder Kraftschluss, zu erreichen.

Bevorzugt sind auf dem Substrat des Biosensors jeweils mindestens eine Arbeitselektrode und mindestens eine Referenzelektrode und/oder mindestens eine Gegenelektrode angeordnet.

Vorzugsweise ist auf mindestens zwei der Außenoberflächen jeweils mindestens eine Elektrode angeordnet. Vorzugsweise können mindestens zwei der Außenoberflächen jeweils mindestens eine Arbeitselektrode und jeweils für beide Außenoberflächen unabhängig voneinander mindestens eine Elektrode ausgewählt aus der Gruppe bestehend aus einer Referenzelektrode und einer Gegenelektrode aufweisen, beispielsweise eine Arbeitselektrode und eine Gegenelektrode oder eine Arbeitselektrode und eine Referenzelektrode oder eine Arbeitselektrode, eine Gegenelektrode und eine Referenzelektrode.

Zusätzlich kann auf mindestens einer der Außenoberflächen, vorzugsweise auf jeder Außenoberfläche, mindestens ein Kontaktierungselement angeordnet sein, also ein Element, welches eine elektrische Kontaktierung ermöglicht. Bevorzugterweise ist jeweils eine Elektrode mit mindestens einem Kontaktierungselement über mindestens eine Leiterbahn miteinander verbunden. Vorzugsweise ist sowohl auf der ersten als auch auf der zweiten Außenoberfläche mindestens ein Kontaktierungselement und/oder mindestens eine Elektrode angeordnet.

In einer weiteren Ausführungsform weisen mindestens zwei der Außenoberflächen jeweils mindestens eine Arbeitselektrode und jeweils mindestens eine Referenzelektrode und/oder Gegenelektrode auf. Es können sich in einer alternativen Ausführungsform auch mehrere Sätze von Arbeitselektroden mit Referenzelektroden und/oder Gegenelektroden befinden. Die Elektroden sind bevorzugt jeweils mit einem Kontaktierungselement verbunden. Das Kontaktierungselement kann sich auf der jeweils gleichen Außenoberfläche befinden oder aber auch auf einer anderen Außenoberfläche als die jeweilige Elektrode.

In verschiedenen Ausführungsformen sind sämtliche Kombinationen von ein oder mehr Elektroden auf einer oder mehreren Außenoberflächen in Kombination mit einem oder mehreren Kontaktierungselementen auf einer oder mehreren Außenoberflächen denkbar. So ist es denkbar, dass auf nur einer Außenoberfläche sich mindestens eine Elektrode bzw. ein Kontaktierungselement befindet. Darüber hinaus gibt es Ausführungsformen, in denen auf nur einer Außenoberfläche mindestens eine Elektrode angeordnet ist, aber auf mindestens zwei Außenoberflächen mindestens jeweils ein Kontaktierungselement angeordnet ist. Genauso ist vorstellbar, dass auf mehreren Außenoberflächen jeweils mindestens eine Elektrode angeordnet ist, sich jedoch nur auf einer Außenoberfläche mindestens ein Kontaktierungselement befindet. In einer weiteren Ausführungsform ist denkbar, dass auf jeder Außenoberfläche mindestens eine Elektrode und mindestens ein Kontaktierungselement vorhanden sind. In einer weiteren Ausführungsform sind nur auf einer Außenoberfläche mindestens eine Elektrode und mindestens ein Kontaktierungselement angebracht. Dies hat den Vorteil, dass bei dem Knickvorgang keine Leiterbahnen geknickt werden. Insbesondere kann der mindestens eine Knickvorgang derart durchgeführt werden, dass sich kein Kontaktierungselement und/oder keine Leiterbahn und/oder keine Elektrode über die Knickstelle hinweg erstreckt. Auch andere Ausgestaltungen sind jedoch möglich.

In einer bevorzugten Ausführungsform sind mindestens ein Kontaktierungselement und mindestens eine Elektrode ausschließlich auf mindestens einer der Außenoberflächen angeordnet. Weiterhin kann sich auf mindestens einer der Außenoberflächen mindestens eine Leiterbahn befinden, die das mindestens eine Kontaktierungselement mit der mindestens einen Elektrode verbindet. Beispielsweise kann das mindestens eine Kontaktierungselement auf der gleichen Außenoberfläche angeordnet sein wie die mindestens eine Elektrode. Zusätzlich kann sich auf mindestens einer weiteren Außenoberfläche mindestens eine weitere Elektrode befinden. Alternativ kann das mindestens eine Kontaktierungselement auf einer anderen Außenoberflächen angeordnet sein als die mindestens eine Elektrode.

In einer Ausführungsform, in der die mindestens zwei Innenflächen nach dem Knicken des Biosensors vorzugsweise nicht miteinander in Kontakt treten, können auch auf einer und/oder beiden Innenflächen wahlweise mindestens eine Elektrode, mindestens ein Kontaktierungselement und/oder mindestens eine Leiterbahn angeordnet sein. Es können auch mehrere Elemente, z.B. mindestens eine Elektrode und mindestens ein Kontaktierungselement, auf einer oder beiden Innenoberflächen angebracht sein. Bevorzugterweise sind die mindestens eine Elektrode und das mindestens eine Kontaktierungselement über eine Leiterbahn miteinander verbunden. Weiterhin können die Elektroden auf der Innenfläche des Substrats auch mit einem oder mehreren Kontaktierungselementen auf einer der Außenoberflächen verbunden sein.

Befindet sich nur eine Elektrode auf einer Außenober- und/oder Innenfläche des Biosensors, so kann diese über eine Leiterbahn mit einem Kontaktierungselement auf der jeweils gegenüberliegenden Innen- bzw. Außenoberfläche verbunden sein. Es sind alle vorstellbaren Kombinationen von Elektroden mit Kontaktierungselementen auf den Innen- bzw. Außenoberflächen bzw. Innen- und Außenoberflächen denkbar.

Je nachdem, wie die Elektroden ausgestaltet sind, ist vorzugsweise jede Elektrode mit einem Kontaktierungselement über mindestens eine Leiterbahn verbunden. Es ist jedoch auch denkbar, dass mehrere Elektroden, die sich auf einer Außenober- bzw. Innenfläche oder auf mehreren Außenober- bzw. Innenflächen befinden können, mit nur einem Kontaktierungselement, das sich auf einer oder auf mehreren Außenober- bzw. Innenflächen erstrecken kann, verbunden sind.

Die Elektroden auf dem Biosensor können unterschiedlicher Art und Ausgestaltung sein. Es kann sich beispielsweise um eine Arbeitselektrode, Referenzelektrode oder Gegenelektrode auf dem Substrat handeln. Die Referenzelektrode kann separat von der Gegenelektrode ausgestaltet sein, kann jedoch auch ganz oder teilweise mit der Gegenelektrode vereint sein. Bevorzugterweise ist mindestens eine Arbeitselektrode und mindestens eine Referenzelektrode und/oder mindestens eine Gegenelektrode bzw. eine Kombination aus Referenz- und Gegenelektrode auf dem Substrat angeordnet. Es können die mindestens eine Arbeitselektrode und die mindestens ein Referenz- bzw. Gegenelektrode sowohl auf einer Außenoberfläche bzw. einer Innenfläche als auch auf zwei verschiedenen Flächen, also einer Außenoberfläche und einer Innenfläche oder zwei Außenoberflächen oder zwei Innenflächen angeordnet sein.

In einer alternativen Anordnung weist der Biosensor mindestens auf zwei der Außenoberflächen jeweils mindestens eine Arbeitselektrode und jeweils mindestens eine Referenzelektrode und/oder Gegenelektrode auf. Zusätzlich können auf den Innenflächen ebenfalls mindestens eine Arbeitselektrode und/oder mindestens eine Referenzelektrode und/oder mindestens eine Gegenelektrode angeordnet sein, sowie sämtliche denkbare Kombinationen aus der mindestens einen Arbeitselektrode, Referenzelektrode und Gegenelektrode.

Alternativ oder zusätzlich können auf dem Biosensor sowohl auf der ersten als auch auf der zweiten Außenoberfläche mindestens ein Kontaktierungselement und/oder mindestens eine Elektrode angeordnet sein. Bevorzugt verbindet mindestens eine Leiterbahn das Kontaktierungselement und die mindestens eine Elektrode.

In einer weiteren bevorzugten Ausführungsform ist die Knickstelle in dem Kontaktierungselement angeordnet. Dabei kann beispielsweise durch die Knickstelle das Kontaktierungselement in zwei Teilbereiche unterteilt werden, wobei beispielsweise ein erster Teilbereich auf einer ersten der Außenoberflächen angeordnet ist und ein zweiter Teilbereich auf einer zweiten Außenoberfläche. Das kann insbesondere dazu führen, dass auf allen Außenoberflächen mindestens ein Kontaktierungselement angeordnet ist. Bevorzugt wird jeweils die mindestens eine Arbeitselektrode auf einer Außenoberfläche mit dem Teil des Kontaktierungselementes verbunden, der sich auf der gleichen Außenoberfläche befindet. Weiterhin bevorzugt sind jeweils alle Arbeitselektroden, alle Referenzelektroden und/oder alle Gegenelektroden mit jeweils einem Kontaktierungselement verbunden. Das Kontaktierungselement kann sich auf nur einer Außenoberfläche erstrecken oder auf mehreren.

In einer bevorzugten Ausführungsform besteht sowohl das Kontaktierungselement als auch die Leiterbahn aus dem gleichen Material. Dieses Material sollte gut elektrisch leitend sein, wie dies z.B. Kupfer, Gold, Silber, Platin oder Palladium oder Legierungen hieraus sind. Alternativ können das Kontaktierungselement und auch die Leiterbahnen aus unterschiedlichen Materialien bestehen. Weitere elektrisch leitende Materialien können elektrisch leitende Polymere oder Materialien mit hohem Carbonanteil sein. Grundsätzlich können alle elektrisch leitenden Materialien hierfür verwendet werden.

Die Elektroden können je nach ihrer Funktion aus unterschiedlichen Materialien bestehen. So kann eine Arbeitselektrode eine oder mehrere Komponenten aufweisen, die eine Umsetzung des zu vermessenden Parameters ermöglichen. Dies können im Fall eines Biosensors vor allem ein oder mehrere Enzyme und/oder Rezeptoren sein. Beispielsweise kann für die Detektion von Glucose als Parameter in der Körperflüssigkeit eine Arbeitselektrode verwendet werden, die beispielsweise Glucoseoxidase (GOD) oder Glucosedehydrogenase (GDh) aufweist. Alternativ können selbstverständlich weitere Parameter wie Lactat, Cholesterin, HbA1C oder andere Parameter aus Blut bzw. interstitieller Flüssigkeit in der Arbeitselektrode umgesetzt und vermessen werden.

Bevorzugt weist der Biosensor mindestens eine biochemische Komponente auf. Als biochemische Komponente werden Moleküle oder Organismen bezeichnet, die befähigt sind bei Kontakt mit einem zu vermessenden Parameter eine Reaktion einzugehen und/oder die befähigt sind, mit einem zu vermessenden Parameter wechselzuwirken. Bei einer Ausführungsform der Erfindung geht die biochemische Komponente bei Kontakt mit dem zu vermessenden Parameter eine chemische Reaktion ein. Ein Parameter ist beispielsweise ein Molekül oder eine Molekülgruppe, die sich in der zu vermessenden Probe befindet. Die biochemischen Komponenten können ausgewählt sein aus der Gruppe bestehend aus einem Enzym, einem Enzym mit Coenzym, einem Rezeptor, einem Organell, einem Bakterium, einem Virus oder mindestens zwei davon. Bei der Reaktion der biochemischen Komponente wird beispielsweise eine optisch, induktiv, magnetisch oder elektrochemisch detektierbare Komponente erzeugt. Diese detektierbare Komponente kann entweder direkt durch ein Detektionsmittel erfasst werden oder wird in einer oder mehreren weiteren Reaktionen in eine detektierbare Komponente umgewandelt.

Als Gegenelektrode wird bevorzugt das gleiche Material wie für die Arbeitselektrode verwendet, wobei vorzugsweise eine reaktive Komponente, wie in diesem Fall das Enzym bzw. die Rezeptoren, nicht auf die für die Gegenelektrode vorgesehene Leiterbahn aufgebracht wird, sondern nur das Trägermaterial aus der Arbeitselektrode ohne das Enzym.

Als Trägermaterial für die biochemische Komponente kann beispielsweise eine elektrisch leitende Komponente, wie beispielsweise eine Carbonpaste verwendet werden.

Die Referenzelektrode kann beispielsweise, wie hinlänglich aus dem Stand der Technik bekannt, aus Ag/AgCl hergestellt werden oder dieses System umfassen. Es können jedoch auch andere Referenzelektroden, wie aus dem Stand der Technik bekannt, eingesetzt werden.

Der Biosensor kann verschiedene geometrische Formen aufweisen, solange er dazu geeignet ist, um in den Körper eines Patienten eingebracht zu werden und über die Kontaktierungselemente mit einem Messgerät in Kontakt zu treten. Bevorzugterweise handelt es sich um einen Biosensor, der nur zu einem Teil in den Körper des Benutzers eingebracht wird. Dieser Teil, der in den Körper des Patienten eingebracht wird, sollte bevorzugterweise die Elektroden aufweisen, um das zu vermessende Körpergewebe in Kontakt mit dem Enzym bzw. dem umzusetzenden Molekül zu bringen.

Um eine möglichst schmerzarme Einbringung des Biosensors zu gewährleisten, weist das Substrat vorzugsweise eine längliche Struktur auf. Unter einer länglichen Struktur ist dabei eine Geometrie zu verstehen, welche eine Längserstreckung und eine Breite aufweist, wobei eine Dimension in der Breite kleiner ist als eine Dimension entlang der Längserstreckung, beispielsweise um mindestens einen Faktor 2, insbesondere um mindestens einen Faktor 3 oder sogar mindestens einen Faktor 5. Insbesondere weist das Substrat vorzugsweise in dem Bereich, in dem es in den Körper insertiert wird, die längliche Form auf.

Der Biosensor kann insbesondere derart ausgestaltet sein, dass sich die mindestens eine Elektrode und/oder mindestens eine Elektrode und das mindestens eine Kontaktierungselement und/oder mindestens ein Kontaktierungselement an einander gegenüberliegenden Enden mindestens einer der Außenoberflächen befinden. Beispielsweise können sich an einer Stirnseite, also einer schmaleren Seite, mindestens einer der Außenoberflächen eine oder mehrere Elektroden und/oder eine oder mehrere Kontaktierungselemente befinden, und an einer gegenüberliegenden Stirnseite ebenfalls mindestens eine Elektrode und/oder mindestens ein Kontaktierungselement. Die oben beschriebene Knickstelle, beispielsweise die mindestens eine Knicklinie, kann sich insbesondere an mindestens einer der Stirnseiten befinden.

Dieser bevorzugte, länglich ausgeformte Teil des Biosensors kann an der Stelle, an welcher sich die mindestens eine Elektrode befindet, insbesondere als Schaft oder Lasche bezeichnet werden, wobei der Schaft bzw. die Lasche mindestens einen Elektrodenbereich umfassen kann oder wobei sich mindestens ein Elektrodenbereich an den Schaft anschließen kann. Der Bereich, in dem sich das mindestens eine Kontaktierungselement befindet, welcher auch als Kontaktierungsbereich bezeichnet werden kann, kann beispielsweise getrennt von dem Elektrodenbereich ausgestaltet sein und kann beispielsweise eine breitere Struktur gegenüber dem Schaft mit dem Elektrodenbereich aufweisen. Dies ist besonders bevorzugt, da die Kontaktierungselemente eine etwas breitere Struktur aufweisen können, um den Biosensor leicht und unkompliziert an ein Messgerät anschließen zu können.

Wie oben dargestellt, weist der Biosensor in einer bevorzugten Ausführungsform ein Substrat mit einer länglichen Struktur auf. Die mindestens eine Elektrode und das mindestens eine Kontaktierungselement können sich insbesondere an einander gegenüberliegenden Enden mindestens einer der Außenoberflächen befinden. Unter einer Anordnung an einem Ende ist dabei eine Anordnung unmittelbar an dem Ende oder eine Anordnung in einem Abstand von nicht mehr als 5 mm von dem Ende, insbesondere von nicht mehr als 3 mm von dem Ende, zu verstehen. Beispielsweise ist eine leicht beabstandete Anordnung der Elektrode im Elektrodenbereich und/oder des Kontaktierungselements am gegenüberliegenden Ende, von dem jeweiligen Ende möglich. Besonders bevorzugt ist es jedoch insbesondere hinsichtlich des Kontaktierungselements, wenn dieses unmittelbar an dem Ende angeordnet ist, beispielsweise an einer Knicklinie.

Die Anordnung von Elektroden und Kontaktierungselementen an gegenüberliegenden Enden des Biosensors kann dazu beitragen, dass der zu insertierende Teil mit den Elektroden deutlich schmaler ausgestaltet werden kann als der Teil mit dem mindestens einen Kontaktierungselement, welcher beispielsweise nicht insertiert wird. Beispielsweise kann ein Ende des Biosensors das mindestens eine Kontaktierungselement tragen, und ein gegenüberliegendes Ende die mindestens eine Elektrode. Diese Geometrie kann die Ausgestaltung des Biosensors vor dem Knicken beeinflussen, je nachdem, welche Form der geknickte Biosensor am Ende haben soll bzw. an welcher Stelle die mindestens eine Knickstelle und damit das verbindende Substratmaterial angeordnet sein sollen.

Das Substrat kann insbesondere, wie oben ausgeführt, mindestens ein Substratmaterial aufweisen. Das Substratmaterial ist bevorzugterweise ein flexibles Material, das sehr dünn ausgestaltet sein kann. Bevorzugterweise ist das Substratmaterial in Folienform zu verwenden, da dies eine leichte Produktion von Band zu Band gewährleistet, und zudem ein leichtes Ausschneiden bzw. Ausstanzen von Biosensorgeometrien gewährleisten kann. Besonders bevorzugt ist das Substrat planar ausgestaltet, so dass es zwei planare Oberflächen aufweist. Es sind jedoch auch alle anderen Formen und Materialien von Substrat vorstellbar. Es können beispielsweise Gießprozesse zur Herstellung von bestimmten Geometrien des Substrats benutzt werden oder Platten, aus denen unterschiedlichste Substratformen herausgeschnitten, gelasert oder gestanzt werden können.

Zur leichten Produktion großer Mengen von Biosensoren kann aus dem bevorzugterweise planaren Substrat eine Vielzahl von Biosensorrohlingen ausgestanzt werden, wobei verbleibende Stege die Vielzahl von Biosensoren miteinander verbinden, so dass sie weiterhin einem oder mehreren Prozessschritten unterzogen werden können, wie beispielsweise dem Bedrucken mit Enzympasten, bevor sie vereinzelt werden. Die Formgebung der Biosensoren aus einem Folienrohling kann jedoch auch nach dem Aufbringen verschiedener Substanzen zur Erzeugung der Kontaktierungselemente und Elektroden erfolgen.

Bevorzugterweise weist das Substratmaterial mindestens ein Polymer auf oder besteht aus mindestens einem Polymer, insbesondere mindestens einem Polyimid. Bevorzugterweise weist das Polymer einen hohen Isolationswiderstand auf, um Kurzschlüsse zwischen den Elektroden zu vermeiden. Besonders bevorzugt ist das Polymer biokompatibel, da es in den Körper des Benutzers eingebracht wird. Alternativ oder zusätzlich kann der Biosensor eine biokompatible Beschichtung, beispielsweise in Form einer biokompatiblen Membran, aufweisen. Weiterhin alternativ oder zusätzlich kann der Sensor eine Membran aufweisen, die eine Diffusionsbarriere für den Analyten, insbesondere Glucose, darstellt.

Um beispielsweise Leckströme zwischen verschiedenen Elektroden zu vermeiden kann über das Substrat mit den aufgebrachten Elektroden und Kontaktierungselementen sowie gegebenenfalls aufgebrachten Leiterbahnen ein Isolierlack aufgebracht werden. Dieser Isolierlack kann darüber hinaus die auf das Substrat aufgebrachten Funktionselemente wie Kontaktierungselemente, Leiterbahnen und Elektroden vor mechanischer Belastung schützen. Zusätzlich kann der Isolierlack bewirken, dass die Funktionselemente wie Kontaktierungselemente, Leiterbahnen und Elektroden, die sich an der Knickstelle befinden ohne mechanische Zug- oder Druckbelastung beispielsweise beim Knickvorgang geknickt werden können. Dabei schließt der Isolierlack die Funktionselemente wie Kontaktierungselemente, Leiterbahnen und Elektroden, zwischen sich und dem Substrat ein. Bevorzugterweise weist der Isolierlack mindestens an der Knickstelle eine Dicke auf, die im Wesentlichen der Dicke des Substrates in der Knickstelle entspricht. Im Wesentlichen der Dicke entsprechen bedeutet hierbei, dass die Dickenabweichung zwischen Isolierlack und Substrat nicht mehr als 20 %, bevorzugt nicht mehr als 10 % beträgt.

In einem weiteren Aspekt der vorliegenden Erfindung wird ein Verfahren zur Herstellung eines Biosensors vorgeschlagen, insbesondere eines Biosensors zum Insertieren in ein subcutanes Gewebe eines Benutzers. Der Biosensor ist ein Biosensor zum qualitativen und/oder quantitativen Nachweis mindestens eines Analyten in einer Körperflüssigkeit sein. Insbesondere kann der Biosensor ein Biosensor gemäß einer oder mehreren der oben beschriebenen Ausgestaltungen sein.

Das vorgeschlagene Verfahren umfasst folgende Schritte, welche vorzugsweise, jedoch nicht notwendigerweise in der dargestellten Reihenfolge durchgeführt werden können. Auch eine andere Reihenfolge ist möglich. Weiterhin können auch zusätzliche, nicht genannte Verfahrensschritte vorgesehen sein. Weiterhin können auch einzelne oder mehrere Verfahrensschritte zeitlich parallel, zeitlich überlappend oder einzeln oder zu mehreren wiederholt durchgeführt werden. Das Verfahren umfasst die folgenden Schritte:
- Bereitstellen mindestens eines flexiblen Substrats, insbesondere eines planaren flexiblen Substrats,
- Aufbringen mindestens eines Kontaktierungselementes und mindestens einer mit dem Kontaktierungselement verbundenen Elektrode auf mindestens eine Oberfläche des Substrats,
- Knicken des Substrats an mindestens einer Knickstelle, so dass die Oberfläche in zumindest zwei miteinander verbundene Außenoberflächen unterteilt wird.

Der Sensor kann insbesondere eine längliche Struktur aufweisen. Das Knicken kann insbesondere in einer Weise erfolgen, ausgewählt aus: einem Knicken parallel zu einer Längserstreckungsachse des Biosensors; einem Knicken quer zu einer Längserstreckungsachse des Biosensors, insbesondere im Wesentlichen senkrecht zur Längserstreckungsachse des Biosensors, beispielsweise mit einer Abweichung zur Senkrechten von nicht mehr als 20°, insbesondere von nicht mehr als 10° und besonders bevorzugt von nicht mehr als 5°.

Die Herstellung kann insbesondere derart erfolgen, dass mehrere Biosensoren im Nutzen hergestellt werden. Beispielsweise können mehrere Biosensoren über einen oder mehrere Stege miteinander verbunden sein, beispielsweise unter Verwendung eines entsprechenden Substratzuschnitts.

Das Knicken kann beispielsweise während eines Vereinzelungsschrittes von mehreren, über einen oder mehrere Stege zusammenhängenden Biosensoren geschehen. Hierzu kann die Folie beispielsweise über eine Metallkante geleitet werden, die an der Knickstelle einen Impuls auf den Biosensor ausübt, so dass das Substrat an der Knickstelle geknickt wird und gleichzeitig von den restlichen Biosensoren abgelöst wird. Das Knicken kann alternativ auch nach dem Vereinzelungsschritt erfolgen.

Als Substrat sollte vorzugsweise ein biokompatibles Material verwendet werden, da mindestens ein Teil des Substrats in Kontakt mit dem Körper des Benutzers tritt. Das Substrat ist bevorzugt planar und wird beispielsweise in Bandform bereitgestellt, kann aber wie bereits erwähnt auch in Plattenform oder sonstiger Form und Geometrie bereitgestellt werden. Es ist auch vorstellbar, dass das Substrat in Form eines runden Drahtes vorliegt, auf den dann die Funktionselemente für den Biosensor aufgebracht werden können. Bevorzugterweise wird das Substrat von einer Seite bestückt, so dass das Aufbringen des mindestens einen Kontaktierungselements und der mindestens einen Elektrode auf mindestens eine Oberfläche des Substrats bewerkstelligt werden kann. Bevorzugterweise wird das Substrat bei dem Produktionsprozess des Biosensors nicht gedreht, sondern bestenfalls horizontal fortbewegt. Es ist jedoch auch vorstellbar, dass das Substrat fixiert wird und das Aufbringen der Funktionselemente über eine bewegliche Aufbringanlage bewerkstelligt wird. Das Aufbringen von den Kontaktierungselementen bzw. Leiterbahnen kann entweder durch Sputteringprozesse oder Tauchprozesse oder Siebdruckprozesse erfolgen.

Werden sowohl auf den Außenober- als auch auf den Innenflächen elektrische Bauelemente aufgebracht, so kann die Produktion auch simultan beidseitig auf den Oberflächen beispielsweise der Bandware erfolgen. Alternativ kann das Substrat auch zunächst von einer Seite behandelt werden und anschließend von der anderen Seite.

Das Aufbringen der Elektroden kann je nach Beschaffenheit des Elektrodenmaterials mit unterschiedlichsten Methoden erfolgen. Diese Methoden sind grundsätzlich hinlänglich im Stand der Technik bekannt. Beispielsweise können lithografische Methoden, Siebdruckverfahren, sonstige Druckverfahren, Pipettieren, chemische Dampfphasenabscheidung, physikalische Dampfphasenabscheidung wie beispielsweise Aufdampfen und/oder Sputtern und/oder Kombinationen der genannten und/oder anderer Verfahren eingesetzt werden.

Bevorzugterweise wird nach dem Aufbringen der verschiedenen Funktionselemente wie Kontaktierungselemente, Leiterbahnen und Elektroden das Substrat an mindestens einer Knickstelle geknickt, so dass die Oberfläche in zumindest zwei miteinander verbundene Außenoberflächen unterteilt wird. Diese Knickstelle kann an unterschiedlichen Orten im Substrat ausgewählt werden. Bevorzugterweise besitzt diese Knickstelle einen Bereich mit weniger Substratmaterial, um den Knickprozess zu erleichtern und gezielt an einer bestimmten Stelle durchführen zu können. Diese Knickstelle mit weniger Substratmaterial kann entweder durch Auskerbung im Substrat, Ausschneiden von Substrat, Wegätzen von Substrat oder Perforieren des Substrats oder sonstige Materialabtragsmethoden hergestellt werden.

Je nachdem, auf welchen Oberflächen nach dem Knickvorgang welche Funktionselemente vorhanden sein sollen und wie der Biosensor schließlich in den Körper eingeführt werden soll, kann das Knicken entweder parallel zu einer Längserstreckungsachse des Biosensors erfolgen oder quer zu einer Längserstreckungsachse des Biosensors, wenn dieser eine längliche Struktur aufweist. Spezielle Ausführungsformen hierzu werden vor allem in der Figurenbeschreibung näher erläutert.

Vorteile der Biosensoren mit geknickter Oberfläche sind vor allem die Möglichkeit, mehr als eine Oberfläche des zu insertierenden Sensors für die Reaktion mit der Körperflüssigkeit bereitzustellen, bei gleichzeitig stark vereinfachtem Herstellungsverfahren. Wie bereits erwähnt, liegt die Vereinfachung zur Herstellung eines Sensors mit Funktionselementen auf mehr als einer Außenoberfläche vor allem an der Möglichkeit, die Funktionselemente vor dem Knickvorgang auf nur eine Oberfläche des Sensors aufbringen zu können. Dies kann beispielsweise durch einseitige Druckverfahren geschehen. Durch das anschließende Knicken des Substrates des Sensors kann der zur Messung zur Verfügung stehende aktive Bereich des Biosensors, in dem sich beispielsweise die Elektroden befinden, um ein Mehrfaches gegenüber einsträngigen Biosensoren erhöht werden. Die für die Messung zur Verfügung stehende Anzahl von Elektroden kann auf diese Weise mindestens verdoppelt werden. Dies ist besonders vorteilhaft, da die Strecke in dem Unterhautfettgewebe (der Subcutis) des Benutzers nur über einen kleinen Bereich von weniger als 10 mm für die Messung von Körperflüssigkeit nutzbar ist, um kein sonstiges Gewebe zu beschädigen. Hierdurch können Biosensoren mit höherer Präzision erreicht werden, da durch jede weitere Elektrode die Genauigkeit und Reproduzierbarkeit eines Biosensors steigt. Durch die Verdoppelung der Elektroden auf der Sensoroberfläche kann bei einer Insertion des Biosensors in einem bevorzugten Winkel von 45° zur Haut eine Erhöhung des Tragekomforts für den Benutzer erreicht werden. Besonders vorteilhaft ist dies gegenüber einem herkömmlichen, längeren Biosensor, der flacher und damit risikoreicher insertiert werden muss, um die gleiche Anzahl von Elektroden in die Haut einzubringen.

Durch das Knicken des Biosensors kann zudem erreicht werden, dass zunächst nur auf einer Oberfläche des Substrates des Biosensors Materialien aufgebracht werden müssen, was zu einem vereinfachten Herstellprozess und damit deutlicher Kostenreduktion führt. Auf diese Weise kann auf einfachem Wege erreicht werden, dass ein Großteil der Substratoberfläche zur Vermessung der Körperflüssigkeit bei insertiertem Biosensor genutzt wird. Zudem existiert eine hohe Variabilität, die Kontaktierung des insertierten Biosensors vorzunehmen, da durch das Knicken an verschiedensten Stellen unter anderem im Kontaktierungselementbereich eine Kontaktierung auf verschiedensten Flächen des Biosensors ermöglicht wird. Auf diese Art und Weise können mit herkömmlichen Herstellverfahren auf nur einer Oberfläche verschiedenste Geometrien von Funktionselementen auf unterschiedlichen Außenoberflächen des Biosensors ermöglicht werden. Dies gewährleistet eine Anpassung an verschiedenste Geometrien von Messgeräten, Insertionssystemen und Bedürfnissen des Benutzers.

In einem weiteren Aspekt der vorliegenden Erfindung wird ein Substratzuschnitt zur Herstellung eines Biosensors vorgeschlagen. Dieser Substratzuschnitt soll zur Herstellung eines Biosensors gemäß einer oder mehreren der oben beschriebenen Ausgestaltungen einsetzbar sein. Der Substratzuschnitt weist mindestens eine Elektrode auf mindestens einer Oberfläche des Substrats und mindestens ein Kontaktierungselement auf, wobei das Kontaktierungselement mit der Elektrode verbunden ist. Das Substrat weist mindestens eine Knickstelle auf, an der das Substrat mindestens zu einem Teil knickbar ist, so dass die Oberfläche in zumindest zwei miteinander verbundene Außenoberflächen unterteilt wird.

Neben den bereits erwähnten Funktionselementen wie dem mindestens einen Kontaktierungselement und der mindestens einen Elektrode sowie optional mindestens einer Leiterbahn, weist der Substratzuschnitt also, wie bereits oben erwähnt, mindestens eine Knickstelle auf. Insbesondere kann die Knickstelle beispielsweise mindestens eine Knicklinie umfassen. Die Knickstelle umfasst mindestens eine Schwächung eines Substratmaterials des Substrats, beispielsweise eine lokale Verdünnung des Substratmaterials. Beispielsweise kann die Knickstelle in einer Weise ausgeformt sein, ausgewählt aus: einem Falz, einer Perforation, einer Knicklinie mit mindestens einer Furche oder Kombinationen hieraus.

Diese Knickstelle kann symmetrisch über dem Substratzuschnitt angeordnet sein, beispielsweise an einander gegenüberliegenden Stellen auf einander gegenüberliegenden Oberflächen des Substratzuschnitts, kann jedoch alternativ auch nur auf einer Seite des Substrates eingebracht sein. Die Knickstelle kann über den Verlauf des Substrats symmetrisch angeordnet sein, so dass das Substrat nach dem Knicken in zwei gleiche Teile unterteilt ist oder aber asymmetrisch, so dass ein Teil des Substrates nach dem Knicken kleiner ist als der andere. Hierdurch können unterschiedlich große oder aber auch gleich große Außenoberflächen erreicht werden. Weiterhin können sich mehr als eine Knickstelle an dem Substrat befinden, so dass der Biosensor an mehr als einer Stelle geknickt werden kann oder an einer Stelle mehrfach geknickt werden kann.

In einer bevorzugten Ausführungsform können zur Bereitstellung der Knickstelle mehrere der Materialverringerungsmaßnahmen in einem Substratzuschnitt verwendet werden. So kann sowohl eine Einkerbung entweder an einer oder an beiden Seiten des Substrates vorliegen und zudem noch eine Perforation im Bereich der Knickstelle vorliegen oder es kann eine Einkerbung zusammen mit einem Falz vorliegen oder es kann eine Einkerbung zusammen mit einer Furche verwendet werden. Außerdem kann die Knickstelle sowohl eine Perforation als auch einen Falz oder eine Furche aufweisen.

In einem weiteren Aspekt der vorliegenden Erfindung wird ein Insertionskit zum Insertieren eines Biosensors in ein subcutanes Gewebe eines Benutzers vorgeschlagen. Dieses Insertionskit umfasst mindestens einen Biosensor gemäß einer oder mehreren der oben beschriebenen Ausgestaltungen. Weiterhin umfasst das Insertionskit mindestens ein Stechelement, beispielsweise mindestens eine Nadel, mindestens eine Flachlanzette, mindestens eine Rundlanzette, mindestens eine Kanüle oder Kombinationen der genannten und/oder anderer Stechelemente.

Dieses Insertionskit soll dazu dienen, dem Benutzer einen leichten Weg zur selbstständigen Insertion des Biosensors bereitzustellen. Hierzu kann der Biosensor in unmittelbare Funktionsnachbarschaft mit dem Stechelement treten. Es sind verschiedene Möglichkeiten der Anordnung des Biosensors zu dem Stechelement vorstellbar.

In einer Ausführungsform kann das Stechelement beispielsweise zwischen den beiden Außenoberflächen des Biosensors angeordnet sein, beispielsweise zwischen zwei Teilen des Substrats, welche durch die Knickstelle voneinander getrennt sind, beispielsweise zwischen den oben beschriebenen Innenoberflächen der Substratteile, welche vorzugsweise parallel zueinander angeordnet sein können.

Beispielsweise kann das Stechelement zwischen den beiden Außenoberflächen in den Biosensor eingeführt werden und so mit dem Biosensor interagieren, dass der Biosensor beim Einstich einer Stechhilfe bzw. des Stechelementes mit in den Körper des Benutzers eingebracht wird, und anschließend bei Herausziehen des Stechelementes im Körper des Benutzers verbleibt. Weiterhin ist vorstellbar, dass der Biosensor beispielsweise in einer Kanüle angeordnet ist und durch diese während des Stechvorganges geschützt ist. Es ist auch vorstellbar, dass eine Außenoberfläche des geknickten Biosensors sich innerhalb der Kanüle befindet, während die zweite Außenoberfläche außerhalb der Kanüle angeordnet ist während des Stechvorgangs.

Eine bevorzugte Ausführungsform ist ein Insertionskit mit einem Stechelement, insbesondere einer Flachlanzette, die zwischen die beiden Außenoberflächen hindurchgeschoben wird und so in Kontakt mit den Innenflächen des Substrats gelangt. Dabei kann das Stechelement, insbesondere die Flachlanzette, beispielsweise eine Aussparung aufweisen, um einen Teil des Substrats, das sich zwischen den beiden Außenoberflächen befindet, aufzunehmen und so ein Mitnehmen des Biosensors während des Stechvorgangs einfach zu ermöglichen. Beim Herausziehen des Stechelements, insbesondere der Flachlanzette, kann der Biosensor vorzugsweise ohne weitere Halterung des Biosensors im Körper des Benutzers belassen werden.

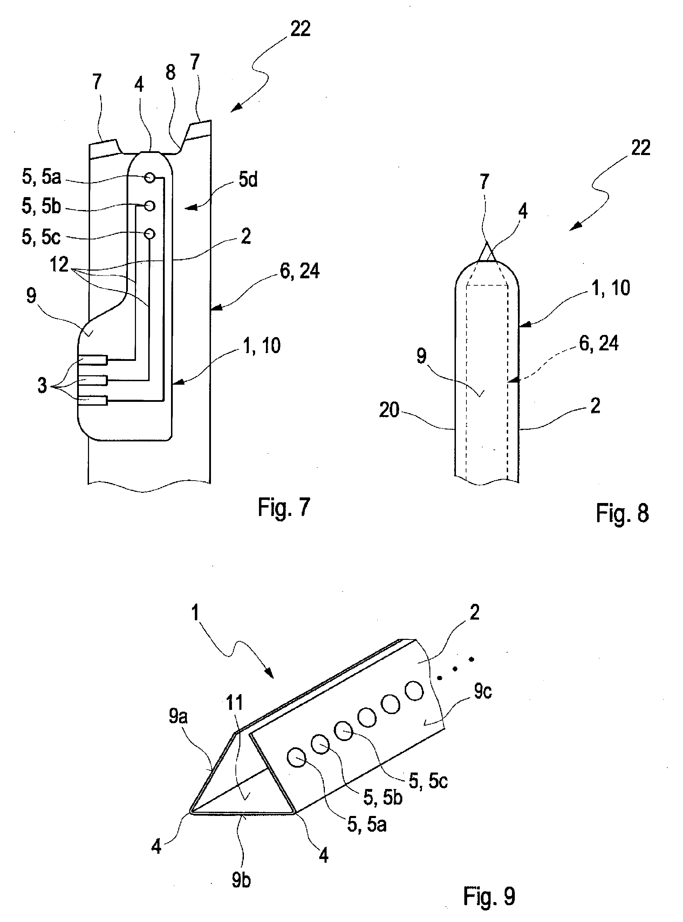

Eine alternative Möglichkeit, das Stechelement, insbesondere die Flachlanzette, zwischen die beiden Außenoberflächen einzuführen, ist ein Durchstechen des Substrats im Bereich der Knickstelle, so dass rechts und links von einer Spitze des Stechelements, beispielsweise einer Lanzettenspitze, der Biosensor von dem Stechelement gehalten wird, während die Spitze frei zum Einstich aus dem Substrat herausragt, wie beispielsweise unten anhand der Figur 8 noch exemplarisch näher erläutert wird.

Beispielsweise wird als Stechelement eine einseitig geschliffene Flachlanzette verwendet, die nach dem Eintritt in das Gewebe des Benutzers entgegen der Schliffseite abgelenkt wird. Dadurch verringert sich der Winkel von Sensor zur Haut kontinuierlich, ohne dass der Insertionswinkel verändert oder verkleinert wird. Die Flachlanzette kann einen Bogen bilden, der einen anatomisch zur Verfügung stehenden Korridor von 2 bis 10 mm unter der Hautoberfläche besser ausnutzt.

Die Verwendung einer Flachlanzette besitzt den Vorteil gegenüber anderen Stechelementen, dass sie weniger Gewebe bei der Insertion verdrängt und somit für den Patienten potenziell weniger schmerzhafte Einstiche erlaubt.

Alternativ oder zusätzlich kann jedoch auch eine runde Lanzette verwendet werden, um die der vorzugsweise flexible Biosensor zumindest teilweise herumgewickelt sein kann. Alternativ kann auch eine Hohlnadel und/oder Kanüle verwendet werden, in welcher der Biosensor aufgenommen sein kann, beispielsweise indem der vorzugsweise flexible Biosensor eingerollt aufgenommen wird. Weiterhin ist vorstellbar, dass der Biosensor ein Polygon ausbildet, welches eigenstabil ist und so beispielsweise in eine Hohlnadel und/oder Hohlkanüle eingebracht werden kann.

Bei der Insertion eines Biosensors mittels des mindestens einen Stechelements können die verschiedensten Geometrien von Biosensoren verwendet werden, wie sie bereits oben erwähnt wurden. So können sich beispielsweise nur auf einer Außenoberfläche des geknickten Biosensors Elektroden und/oder Kontaktierungselemente befinden oder auf beiden Außenoberflächen. Darüber hinaus oder alternativ können sich auf der Innenseite des Biosensors ebenfalls Elektroden und/oder Kontaktierungselemente befinden.

Allgemein sind im Rahmen der vorliegenden Erfindung die folgenden Ausführungsformen besonders bevorzugt:
Ausführungsform 1: Biosensor zum Insertieren in ein subcutanes Gewebe eines Benutzers, wobei der Biosensor eingerichtet ist zum qualitativen und/oder quantitativen Nachweis mindestens eines Analyten in einer Körperflüssigkeit, wobei der Biosensor mindestens ein flexibles Substrat und mindestens eine Elektrode auf mindestens einer Oberfläche des Substrats und mindestens ein Kontaktierungselement aufweist, wobei das Kontaktierungselement mit der Elektrode verbunden ist, wobei das Substrat mindestens eine Knickstelle aufweist, an der das Substrat mindestens zu einem Teil geknickt ist, so dass die Oberfläche in zumindest zwei miteinander verbundene Außenoberflächen unterteilt ist.
Ausführungsform 2: Biosensor nach der vorhergehenden Ausführungsform, dadurch gekennzeichnet, dass auf mindestens zwei der Außenoberflächen jeweils mindestens eine Elektrode angeordnet ist.
Ausführungsform 3: Biosensor nach einer der vorhergehenden Ausführungsformen, dadurch gekennzeichnet, dass die Außenoberflächen mindestens zwei zueinander im Wesentlichen parallel angeordnete Außenoberflächen aufweisen.
Ausführungsform 4: Biosensor nach einer der vorhergehenden Ausführungsformen, dadurch gekennzeichnet, dass durch die Knickstelle das Substrat in mindestens zwei miteinander verbundene Substratteile unterteilt wird, wobei die Substratteile mindestens zu einem Teil miteinander verbunden sind, insbesondere verklebt sind.
Ausführungsform 5: Biosensor nach einer der vorhergehenden Ausführungsformen, dadurch gekennzeichnet, dass die mindestens eine Elektrode mindestens eine Arbeitselektrode und/oder mindestens eine Referenzelektrode und/oder mindestens eine Gegenelektrode umfasst.
Ausführungsform 6: Biosensor nach einer der vorhergehenden Ausführungsformen, dadurch gekennzeichnet, dass sowohl auf der ersten als auch auf der zweiten Außenoberfläche mindestens ein Kontaktierungselement und/oder mindestens eine Elektrode angeordnet sind.
Ausführungsform 7: Biosensor nach einer der vorhergehenden Ausführungsformen, dadurch gekennzeichnet, dass die Knickstelle in dem Kontaktierungselement angeordnet ist.
Ausführungsform 8: Biosensor nach einer der vorhergehenden Ausführungsformen, dadurch gekennzeichnet, dass zwischen dem Kontaktierungselement und der Elektrode mindestens eine Leiterbahn angeordnet ist.
Ausführungsform 9: Biosensor nach einer der vorhergehenden Ausführungsformen, dadurch gekennzeichnet, dass das Substrat eine längliche Struktur aufweist und dass sich die eine Elektrode und das Kontaktierungselement an einander gegenüberliegenden Enden mindestens einer der Außenoberflächen befinden.
Ausführungsform 10: Substratzuschnitt zur Herstellung eines Biosensors nach einer der vorhergehenden Ausführungsformen, wobei der Substratzuschnitt mindestens ein flexibles Substrat aufweist und mindestens eine Elektrode auf mindestens einer Oberfläche des Substrats und mindestens ein Kontaktierungselement aufweist, wobei das Kontaktierungselement mit der Elektrode verbunden ist, wobei das Substrat mindestens eine Knickstelle aufweist, an der das Substrat mindestens zu einem Teil knickbar ist, so dass die Oberfläche in zumindest zwei miteinander verbundene Außenoberflächen unterteilt wird, wobei die Knickstelle (4) mindestens eine Schwächung eines Substratmaterials des Substrats (2) umfasst.
Ausführungsform 11: Substratzuschnitt nach der vorhergehenden Ausführungsform, wobei die Knickstelle in einer Weise ausgeformt ist ausgewählt aus: einem Falz, einer Perforation, einer Knicklinie mit einer Furche oder Kombinationen hieraus.
Ausführungsform 12: Insertionskit zum Insertieren eines Biosensors in ein subcutanes Gewebe eines Benutzers, umfassend mindestens einen Biosensor nach einer der vorhergehenden, einen Biosensor betreffenden Ausführungsformen, weiterhin umfassend ein Stechelement.
Ausführungsform 13: Insertionskit nach der vorhergehenden Ausführungsform, wobei das Stechelement ausgewählt ist aus: einer Nadel, einer Lanzette, einer Flachlanzette, einer Rundlanzette oder einer Kanüle.
Ausführungsform 14: Insertionskit nach einer der vorhergehenden beiden Ausführungsformen, wobei das Stechelement zwischen den beiden Außenoberflächen angeordnet ist.
Ausführungsform 15: Verfahren zur Herstellung eines Biosensors zum qualitativen und/oder quantitativen Nachweis mindestens eines Analyten in einer Körperflüssigkeit, insbesondere eines Biosensors nach einer der vorhergehenden, einen Biosensor betreffenden Ausführungsformen, mit den Schritten:
   - Bereitstellen mindestens eines flexiblen Substrats,
   - Aufbringen mindestens eines Kontaktierungselementes und mindestens einer mit dem Kontaktierungselement verbundenen Elektrode auf mindestens eine Oberfläche des Substrats,
   - Knicken des Substrats an mindestens einer Knickstelle, so dass die Oberfläche in zumindest zwei miteinander verbundene Außenoberflächen unterteilt wird.
Ausführungsform 16: Verfahren nach der vorhergehenden Ausführungsform, wobei der Biosensor eine längliche Struktur aufweist und das Knicken in einer Weise erfolgt, ausgewählt aus: einem Knicken parallel zu einer Längserstreckungsachse des Biosensors; einem Knicken quer zu einer Längserstreckungsachse des Biosensors.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche beziehungsweise hinsichtlich ihrer Funktionen einander entsprechende Elemente.

Im Einzelnen zeigen:
- Figur 1:: eine Aufsicht auf einen ungefalteten Biosensor mit Kontaktierungselementen und Elektroden sowie einer Knicklinie;
- Figur 2a:: eine Aufsicht auf einen Teil eines Biosensors mit einer Knicklinie, die eine Auskerbung vorsieht;
- Figur 2b:: eine Aufsicht auf einen ungeknickten Biosensor im Bereich einer Knickstelle mit einer Perforation in einer Knickstelle;
- Figur 2c:: eine Seitenansicht eines ungeknickten Biosensors mit einer Knickstelle mit einer beidseitigen Furche;
- Figur 3a:: eine Aufsicht auf einen Biosensor mit einer Knickstelle im Bereich von Kontaktierungselementen;
- Figur 3b:: eine Darstellung des Biosensors gemäß Figur 3a nach einem Knicken;
- Figur 4a:: eine Aufsicht auf einen Biosensor mit seitlicher Knickstelle;
- Figur 4b:: eine Darstellung des Biosensors aus Figur 4a nach einem Knicken;
- Figur 5a:: eine Darstellung eines ungeknickten Biosensors mit längsseitiger Knickstelle;
- Figur 5b:: eine Darstellung des Biosensors aus Figur 5a nach einem Knicken;
- Figur 6a:: eine Darstellung eines ungeknickten Biosensors mit einer Knickstelle in einem Elektrodenbereich;
- Figur 6b:: eine Darstellung des Biosensors aus Figur 6a mit Knickstelle im Elektrodenbereich;
- Figur 7:: eine Darstellung eines geknickten Biosensors im Elektrodenbereich mit einer eingeschobenen Lanzette;
- Figur 8:: eine Darstellung eines geknickten Biosensors mit eingeschobener Lanzette, wobei die Lanzettenspitze durch die Knickstelle hindurchgeführt wird; und
- Figur 9:: eine Darstellung eines Biosensors mit mehreren Knickstellen.

### Ausführungsbeispiele

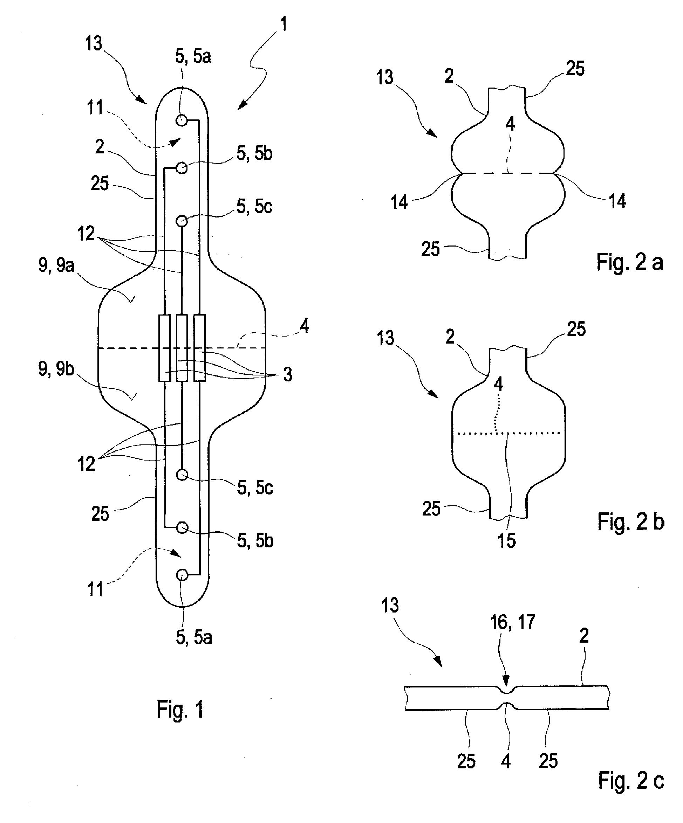

In Figur 1 ist schematisch ein Biosensor 1 in einem ungeknickten Ausgangszustand gezeigt, welcher gleichzeitig auch ein Ausführungsbeispiel für einen Substratzuschnitt 13 darstellt. Der Biosensor 1 weist ein Substrat 2 auf, auf dessen einer Oberfläche 9 Kontaktierungselemente 3 aufgebracht sind, die über Leiterbahnen 12 mit Elektroden 5, wie beispielsweise einer Arbeitselektrode 5a, einer Gegenelektrode 5b bzw. einer Referenzelektrode 5c, auf einer ersten Außenoberfläche 9a des Substrats 2 verbunden sind. Auf einer zweiten Außenoberfläche 9b des Substrats 2 sind wiederum über Leiterbahnen 12 die Kontaktierungselemente 3 mit weiteren Elektroden 5 verbunden, die wiederum aus einem Elektrodensatz aus Arbeitselektrode 5a, Gegenelektrode 5b und Referenzelektrode 5c bestehen können. Es ist ebenfalls vorstellbar, dass auf der ersten Außenoberfläche 9a und der zweiten Außenoberfläche 9b Kontaktierungselemente 3 angebracht sind, die nicht miteinander verbunden sind. Es können noch weitere Elektroden 5 und Kontaktierungselemente 3 auf dem Biosensor 1 angebracht sein, die hier jedoch nicht gezeigt sind.

Im Bereich der Kontaktierungselemente 3 ist eine Knickstelle 4 angebracht, über die der Biosensor 1 nach dem Aufbringen der Kontaktierungselemente 3 bzw. der Elektroden 5 geknickt werden kann, so dass das Substrat 2 in zwei Substratteile 25 unterteilt wird. Wenn der Biosensor 1 um die Knickstelle 4 geknickt wird, weist der Biosensor 10 zwei miteinander verbundene Außenoberflächen (9a und 9b) auf, die jeweils einen Bereich mit Kontaktierungselementen 3 und Elektroden 5 besitzen. Dabei liegen bevorzugterweise die Kontaktierungselemente 3 auf den beiden Außenoberflächen 9a, 9b an einem Ende des geknickten Biosensors 10. In diesem Beispiel in Figur 1 sind die Kontaktierungselemente 3 weiterhin jeweils miteinander verbunden, da die Knicklinie 4 quer zu den Kontaktierungselementen 3 verläuft und durch diese hindurch ausgerichtet ist. Dies ist jedoch nicht zwingend notwendig. Es können auch nur auf einer Außenoberfläche 9a, 9b, 9c Kontaktierungselemente 3 angebracht sein oder auf mehreren Außenoberflächen 9a, 9b, 9c von einander separierte Kontaktierungselemente 3. Genauso liegen nach dem Knicken des Biosensors 1 um die Knickstelle 4 die Elektroden 5 (im Speziellen die Arbeitselektrode 5a, die Gegenelektrode 5b und die Referenzelektrode 5c) auf der ersten Außenoberfläche 9a, in die gleiche Richtung des länglichen Schaftes bzw. der länglichen Lasche 20 weisend wie die Elektroden 5, 5a, 5b, 5c der zweiten Außenoberfläche 9b. Folglich liegen nach dem Knicken des Biosensors 1 um die Knickstelle 4 die Kontaktierungselemente 3 und die Elektroden 5 des Elektrodenbereiches 5d der beiden Außenoberflächen 9a und 9b an gegenüberliegenden Enden des geknickten Biosensors 10. Auf diese Weise kann gewährleistet werden, dass nicht Kotaktelemente 3 und Elektroden 5 gleichzeitig in einen Körper des Benutzers insertiert werden. Bevorzugterweise bleiben die Kontaktierungselemente 3 außerhalb den Körpers, während die Elektroden 5 mit der Körperflüssigkeit des Benutzers in Kontakt treten.

Weiterhin ist vorstellbar, dass neben den Elektroden 5 auf den beiden Außenoberflächen 9a und 9b auch Elektroden 5 auf den Innenflächen des Substrats 2 angebracht sind. Diese Innenflächen, welche in Figur 1 symbolisch mit den Bezugsziffern 11 bezeichnet sind, sind auf der, der Oberfläche 9 gegenüberliegenden Rückseite des Substrats 2 in der Aufsicht gemäß Figur 1 angeordnet. Bei dieser Ausführungsform, die hier nicht gezeigt ist, ist der Biosensor 1 vorzugsweise um weniger als 180 ° um die Knickstelle 4 geknickt, so dass die Elektroden 5 auf der Innenfläche 11 ebenfalls für Körperflüssigkeit des Benutzers im insertierten Zustand zugänglich sind. Die Elektroden 5 können ebenfalls über die Kontaktierungselemente 3 auf den Außenoberflächen 9a und 9b verbunden sein, oder über weitere Kontaktierungselemente 3 auf den Innenflächen 11 oder einer der Außenoberflächen 9a oder 9b.

Die Knickstelle 4 kann auf verschiedene Arten in den Biosensor 1 eingebracht werden. Zum einen kann das Substrat 2 selbst aus einem sehr dünnen knickbaren Material gefertigt sein, so dass der Biosensor 1 sehr leicht an der Knickstelle 4 geknickt werden kann. Zum anderen kann der Biosensor 1, wie in Figur 2a gezeigt, mindestens eine Einkerbung 14 in dem Substrat 2 aufweisen, so dass eine leichte Knickung in diesem Bereich des Biosensors 1 erreichbar gemacht wird. Alternativ oder zusätzlich zu der Einkerbung 14 aus Figur 2a kann mindestens eine Perforation 15 in dem Substrat 2 an der Knickstelle 4 verwendet werden, wie in Figur 2b gezeigt. Eine solche Perforation 15 kann beispielsweise punktuelle Materialabtragungen des Substrats 2 in der Knickstelle 4 aufweisen. Aufgrund dieser Materialfehlstellen ist das Substrat 2 bzw. der Biosensor 1 an dieser Stelle labiler und damit leichter knickbar.

Eine weitere Möglichkeit, eine Knickstelle 4 des Biosensors 1 leichter knickbar als das restliche Substrat 2 des Biosensors 1 zu generieren, ist, den Biosensor 1 an der Knickstelle 4 aus dünnerem bzw. weniger Material herzustellen als das restliche Substrat 2, wie dies in einer Seitenansicht des Substrats 2 in Figur 2c gezeigt ist. Diese Art von Materialminderung im Knickbereich 4 des Biosensors 1 kann aufgrund von Anpassungen des Herstellungsprozesses geschehen, wie dies in Figur 2c dargestellt ist, indem weniger Material in der Knickstelle 4 bereitgestellt wird. Beispielsweise kann auf diese Weise eine Furche 16 oder ein Falz 17 entstehen, wobei eine Furche 16 beispielsweise eine beliebige einseitige oder beidseitige Verdünnung des Materials des Substrats 2 umfassen kann, mit grundsätzlich beliebigem Querschnitt, beispielsweise einem dreieckigen oder runden Querschnitt, beispielsweise in Form einer Rillung und/oder Nutung.

In der Variante aus Figur 2a wird beispielsweise die Einkerbung 14 vor und/oder während und/oder nach dem Herstellprozess des Biosensors 1 an der Knickstelle 4 eingebracht werden, beispielsweise durch Ausstanzen, Ausschneiden oder auf chemischem Wege, z.B. durch Ätzen. Dies kann auf beiden Seiten des Substrates 2, wie in Figur 2a dargestellt, vorgenommen werden oder auch nur auf einer Seite des Substrates 2.

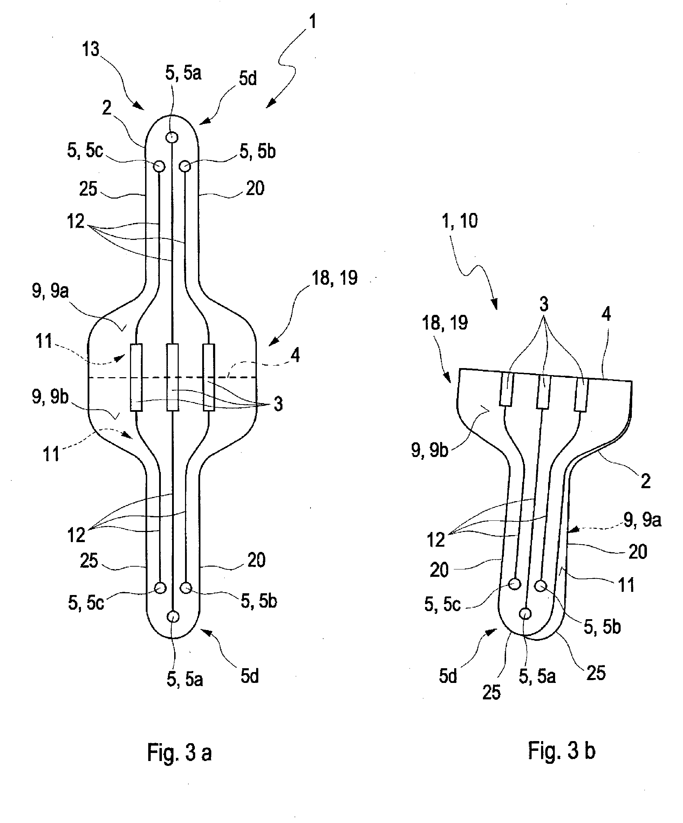

In den Figuren 3a bis 6b werden vier bevorzugte Varianten beschrieben, welche grundsätzlich auch kombinierbar sind und welche sich im Wesentlichen darin unterscheiden, an welchen Stellen des Biosensors 1 die Knickstelle 4 angeordnet sein kann, um nach Knickung an der Knickstelle 4 einen geknickten Biosensor 1 mit zwei miteinander verbundenen Außenoberflächen 9a, 9b zu generieren.

In Figur 3a ist ein Biosensor 1 wie aus Figur 1 dargestellt, der als Grundkörper ein Substrat 2 aufweist, wobei das Substrat 2 länglich geformt ist und zwei annähernd spitz oder abgerundet zulaufende Enden aufweist. In der Mitte des Biosensors 1 ist ein bauchiger Abschnitt mit einer Verbreiterung 18 gezeigt, der in diesem Fall die Knickstelle 4 aufweist. Die Oberfläche 9 wird durch die Knickstelle 4 in die beiden Außenoberflächen 9a und 9b unterteilt, wobei die beiden Außenoberflächen 9a und 9b die Oberflächen darstellen, auf denen mindestens ein Kontaktierungselement 3 und eine Elektrode 5 aufgebracht sind, wobei die beiden Außenoberflächen 9a und 9b nach dem Knicken um die Knickstelle 4 des Biosensors 1 nach außen weisen, wie dies in Figur 3b dargestellt ist. Der geknickte Biosensor 1 ist symbolisch mit der Bezugsziffer 10 bezeichnet.

In dem besonderen Fall des Biosensors 1 aus Figur 3a befinden sich beispielsweise drei Kontaktierungselemente 3 auf der Oberfläche 9 des ungeknickten Biosensors 1. Durch die Kontaktierungselemente 3 verläuft die Knickstelle 4, so dass nach dem Knickvorgang sowohl auf der ersten Außenoberfläche 9a als auch auf der zweiten Außenoberfläche 9b ein Teil der Kontaktierungselemente 3 vorhanden sind, wie in Figur 3b gezeigt. Jedes dieser Kontaktierungselemente 3 ist sowohl auf der ersten Außenoberfläche 9a als auch auf der zweiten Außenoberfläche 9b über Leiterbahnen 12 mit jeweils einer Elektrode 5 verbunden, so dass sowohl an dem einen spitzen Ende des Biosensors 1 aus Figur 3a drei Elektroden 5, nämlich eine Arbeitselektrode 5a, eine Gegenelektrode 5b und eine Referenzelektrode 5c, vorhanden sind, als auch auf der zweiten Außenoberfläche 9b, wie dies in Figur 3b schematisch dargestellt ist.

Wie aus Figur 3b zu erkennen ist, weist der geknickte Biosensor 10 an einem Ende einen Bereich mit den Kontaktierungselementen 3 auf. Dieser Bereich ist etwas verbreitert gegenüber dem Schaft bzw. Lasche 20, an dem die Leiterbahnen 12 entlang laufen, bis sie an dem spitzen Ende des geknickten Biosensors 10 in den Elektroden 5, 5a, 5b und 5c enden. Diese Aufteilung von Kontaktierungselementen 3 und Elektroden 5 ist besonders bevorzugt, da für die Benutzung des Biosensors 10 nur der Elektrodenbereich 5d mit den Elektroden 5 zusammen mit einem Teil der Leiterbahn 12 in den Körper des Patienten eingebracht werden soll, um eine Kontaktierung der Elektroden 5 mit Körperflüssigkeit zu gewährleisten. Der gegenüberliegende Kontaktierungsbereich 19 mit den Kontaktierungselementen 3 wird bevorzugterweise nicht in den Körper eingebracht, sondern stellt die Möglichkeit zur Kontaktierung des Biosensors 10 mit einem elektronischen Messgerät dar. Da die Kontaktierungselemente 3 etwas breiter hergestellt werden als die Leiterbahnen 12 und Elektroden 5, wird das Substrat 2 des Biosensors 1, 10 an diesem Kontaktierungsbereich 19, wie oben ausgeführt, etwas breiter ausgearbeitet und mit der Verbreiterung 18 ausgestattet, um eine möglichst einfache Kontaktierung mit einem Messgerät vornehmen zu können. Diese Verbreiterung 18 des Biosensors 1, 10, welche grundsätzlich in diesem oder auch in anderen Ausführungsbeispielen einsetzbar ist, ist auch deshalb von Vorteil, weil mit dieser Verbreiterung 18 verhindert wird, dass der Sensor 10 in Gänze in den Körper des Patienten eindringen kann.

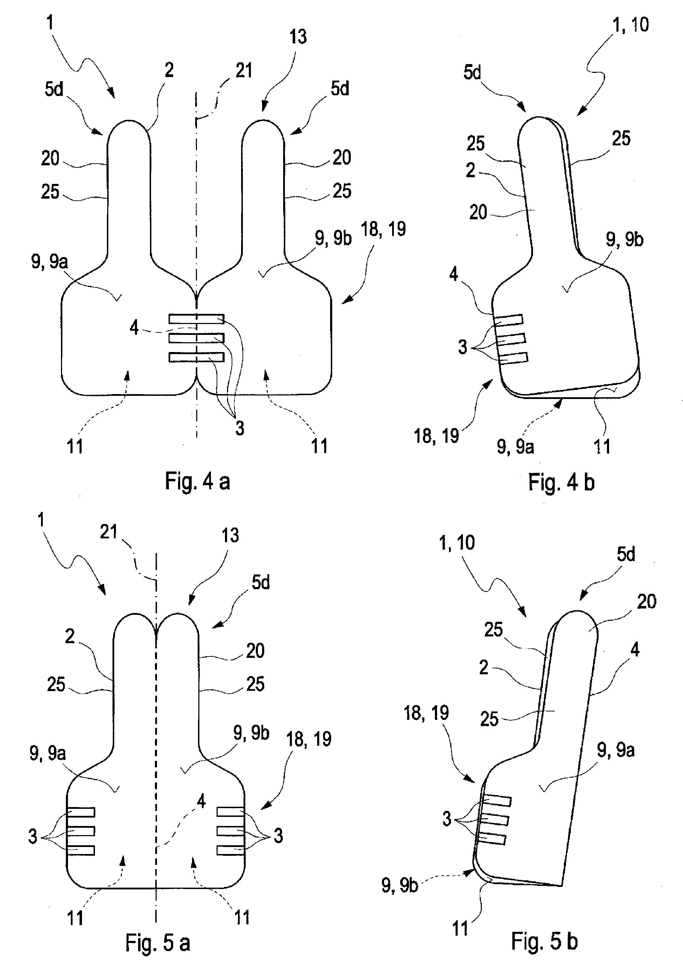

In den Figuren 4a und 4b ist wiederum ein Biosensor 1 vor dem Knickvorgang (Figur 4a) und derselbe Biosensor 1, 10 nach dem Knickvorgang (Figur 4b) dargestellt. Auch hier weist der Biosensor 1, 10 eine schmalere Lasche 20 auf, die bevorzugterweise die Elektroden 5 und optional Leiterbahnen 12 aufnimmt (in den Figuren 4a und 4b nicht dargestellt, Anordnung beispielsweise gemäß den Figuren 3a und 3b), und eine Verbreiterung 18, die mit den Kontaktierungselementen 3 versehen ist. Im Gegensatz zu dem Biosensor 1 aus Figur 3a befindet sich die Knicklinie 4 nicht quer zu einer Längserstreckungsachse 21 des Biosensors 1, sondern längs zu dieser Längserstreckungsachse 21. Im Gegensatz zu dem in Figur 4a dargestellten Beispiel kann der Biosensor 1 in diesem oder auch in anderen Ausführungsbeispielen auch nur auf einer der beiden Außenoberflächen 9a bzw. 9b Kontaktierungselemente 3 aufweisen, so dass nach dem Knickvorgang nur auf einer der beiden Außenoberflächen 9a oder 9b Kontaktierungselemente 3 vorliegen. Durch die Anordnung der Knicklinie bzw. Knickstelle 4 parallel zu den Laschen 20 des Biosensors 1 hängt der Biosensor 10 nach dem Knickvorgang nicht an einer oberen oder unteren Kante des Biosensors 10 mit seinen Außenoberflächen 9a und 9b zusammen, sondern an einer seitlichen Kante der beiden Oberflächen 9a und 9b. Dabei bilden die beiden den Außenoberflächen 9a und 9b gegenüberliegenden Seiten des Substrats 2 nach dem Knickvorgang die Innenflächen 11 des Substrats 2, wie auch in den Figuren 3b, 4b und 6b gezeigt.

Eine alternative geometrische Ausgestaltung des Biosensors 1 ist in den Figuren 5a und 5b gezeigt, bei denen der Biosensor 1 vor dem Knickvorgang nur eine längliche Ausdehnung in eine Richtung aufweist. Eine Lasche 20 erstreckt sich dabei länglich in einer Richtung einer Längserstreckungsachse 21, wobei auf einem Endbereich der Lasche 20 Elektroden 5 angeordnet werden können, sowie gegebenenfalls eine oder mehrere Leiterbahnen 12, welche in den Figuren 5a und 5b nicht dargestellt sind. Weiterhin weist der Biosensor 1 wiederum eine Verbreiterung 18 auf, welche Kontaktierungselemente 3 aufnimmt. Eine Knickstelle 4, welche beispielsweise wiederum als Knicklinie ausgestaltet sein kann, läuft in diesem Ausführungsbeispiel vorzugsweise symmetrisch durch die Bereiche der Verbreiterung 18 mit den Kontaktierungselementen 3 und die schmalere Lasche 20 mit einem Elektrodenbereich 5d des Biosensors 1, 10. Für den Knickvorgang liegt die Knickstelle 4 vorzugsweise in der länglich verlaufenden, vorzugsweise spitz oder abgerundet zulaufenden Lasche 20 und verläuft durch diese hindurch bis zur gegenüberliegenden Verbreiterung 18, in welcher die Kontaktierungselemente 3 vorliegen.

Beim Knickvorgang des Biosensors 1 aus Figur 5a wird der Sensor 1 entlang der Knicklinie 4 umgeknickt, so dass beispielsweise die Lasche 20 mit den Elektroden 5 auf der einen Seite angeordnet sein kann. Weiterhin kann der Knickvorgang derart erfolgen, dass sich ein Kontaktierungsbereich 19 nur in eine Richtung erstreckt, mit den Kontaktierungselementen 3. In den Biosensoren 10 aus den Figuren 4b und 5b erfolgt vorzugsweise eine seitliche Kontaktierung des Biosensors 10, was zu unterschiedlichen Geometrien einer Messanordnung innerhalb eines Sensorsystems führen kann.

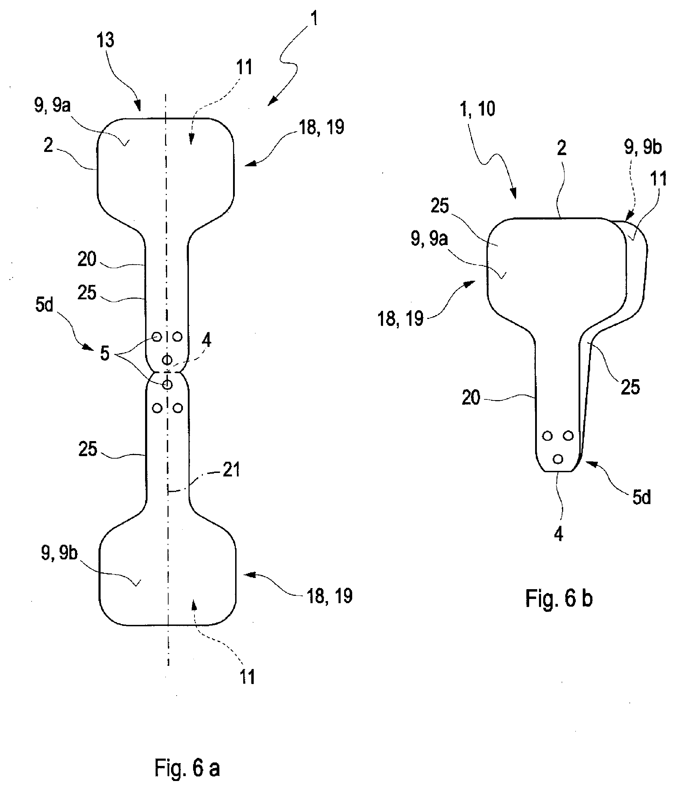

In einem weiteren, in Figur 6a dargestellten Beispiel eines Biosensors 1 sind schematisch nur Elektroden 5 in der Mitte einer länglichen Lasche 20 angedeutet, wobei die längliche Lasche 20 an ihren einander gegenüberliegenden Enden jeweils in einer Verbreiterung 18 rechts und links einer Längserstreckungsachse 21 mündet. In einer oder beiden dieser Verbreiterungen 18 können bevorzugterweise Kontaktierungselemente 3 angeordnet sein. Wird nun entlang einer in diesem Ausführungsbeispiel exemplarisch senkrecht zur Längserstreckungsachse 21 ausgerichteten Knickstelle 4 des Biosensors 1 die zweite Außenoberfläche 9b nach hinten geklappt, so erreicht der Biosensor 1 seine Endstellung in Form des geknickten Biosensors 10 wie in Figur 6b gezeigt. Dabei weist der geknickte Biosensor 10, wie auch schon in den Varianten aus Figur 3b, 4b und 5b gezeigt, einen spitzer oder abgerundet zulaufenden Elektrodenbereich 5d auf, der über die längliche Lasche 20 mit der Verbreiterung 18 und dem Kontaktierungsbereich 19 mit den Kontaktierungselementen 3 verbunden ist. In dem speziellen Fall der Knicklinienanordnung aus Figur 6a sind die beiden Laschen 20 des geknickten Biosensor 10 aus Figur 6b in dem Elektrodenbereich 5d zwischen seinen Außenoberflächen 9a und 9b miteinander verbunden.

Für all diese Ausführungsformen aus den Figuren 1 bis 6b können sowohl auf beiden Außenoberflächen 9a und 9b Kontaktierungselemente 3 und Elektroden 5 sowie die Leiterbahnen 12 zwischen den Kontaktierungselementen 3 und Elektroden 5 vorgesehen sein, oder aber auch nur auf einer der beiden Außenoberflächen 9a bzw. 9b.

Um einen solchen Biosensor 1, 10 aus den Figuren 3b, 4b, 5b oder 6b in den Körper eines Patienten einzubringen, kann, wie in Figur 7 gezeigt, beispielsweise ein Insertionskit 22 verwendet werden, welches neben mindestens einem Biosensor 1, 10 mindestens ein Stechelement 24 umfasst. Dieses Stechelement 24 ist exemplarisch in den Figuren 7 und 8 in Form einer Lanzette 6 gezeigt. Das Stechelement 24 kann insbesondere zwischen die beiden Außenoberflächen 9a und 9b bzw. Innenflächen 11 des geknickten Biosensors 10 geschoben werden, so dass beispielsweise das Stechelement 24 mit den Innenflächen 11 des Substrats 2 in Kontakt tritt.

In Figur 7 ist eine Lanzette 6 mit einem länglichen Schliff an einer Lanzettenspitze 7 gezeigt, wobei dieser Schliff eine Aussparung 8 in der Lanzettenspitze 7 aufweist, um den geknickten Biosensor 10 an seiner Knickstelle 4 aufnehmen zu können. Diese Ausnehmung 8 weist vorzugsweise keinen Schliff auf, um den Sensor 10 nicht zu verletzen. Da in dem Biosensor 10 an der Knickstelle 4 die beiden Außenoberflächen 9a und 9b miteinander verbunden sind, kann der Biosensor 1, 10 durch Einstechen der Lanzette 6 in den Körper des Patienten ohne weitere Hilfsmittel eingebracht werden und verbleibt im Körper des Patienten nach Herausziehen der Lanzette 6, vorzugsweise ohne weitere Utensilien.

Eine alternative Ausgestaltung des Insertionskits 22 mit einer alternativen Anordnung einer Lanzette 6 und eines geknickten Biosensors 10 ist in Figur 8 gezeigt. Hier verläuft die Lanzette 6 innerhalb des geknickten Biosensors 10, beispielsweise innerhalb einer Lasche 20 des geknickten Biosensors 10. Die Lanzette 6 kann insbesondere wiederum zwischen den beiden Außenoberflächen 9a und 9b in Kontakt mit den Innenflächen 11 des Substrats 2 des Biosensors 10 verlaufen. Die Lanzette 6 aus Figur 8 besitzt beispielsweise eine spitz zulaufende Lanzettenspitze 7, die durch das Substrat 2 des Biosensors 10 an der Knickstelle 4 hindurchgestochen wird, so dass der Biosensor 10 ebenfalls mit der Lanzette 6 zusammen in den Körper des Benutzers eingebracht werden kann.

Alternativ zu den beiden in Figur 7 und 8 gezeigten Möglichkeiten, den Biosensor 1, 10 in den Körper des Benutzers einzubringen, sind weitere Möglichkeiten denkbar. Insbesondere kann das Stechelement 24 eines Insertionskits 22 auf andere Weise ausgestaltet sein. Beispielsweise kann eine Hohlkanüle vorgesehen sein, in die die Lasche 20 des Biosensors 1, 10 und/oder ein anderer Teil des Biosensors 1, 10 so eingebracht wird, dass diese während des Einstichvorgangs geschützt ist.

Eine weitere Ausgestaltung des Biosensors 10 nach einem oder mehreren Knickvorgängen ist in Figur 9 dargestellt. Hier ist schematisch ein Biosensor 10 mit zwei Knicklinien 4 dargestellt, der drei Außenoberflächen 9a, 9b, 9c aufweist. Durch die gewinkelte Anordnung der drei Außenoberflächen 9a, 9b, 9c zueinander, weist der Biosensor 10 in Figur 9 eine dreidimensionale Ausgestaltung mit polygonalem Querschnitt, hier exemplarisch einem dreieckigen Querschnitt, auf. Exemplarisch werden in Figur 9 mehrere Elektroden 5 auf einer dritten Außenoberfläche 9c gezeigt. Die hier mit der Bezugsziffer 5 bezeichneten Elektroden können wahlweise eine oder mehrere Arbeitselektroden 5a, eine oder mehrere Gegenelektroden 5b und/oder eine oder mehrere Referenzelektroden 5c sein oder umfassen. Auch eine andere Ausgestaltung der Elektroden 5 ist jedoch möglich, beispielsweise hinsichtlich der Anzahl, der Verwendung oder der Anordnung der Elektroden 5 von der Ausgestaltung in Figur 9 abweichende Ausgestaltung. Die Elektroden 5 sind beispielsweise, wie bereits oben beschrieben, aber hier nicht dargestellt, mit mindestens einem Kontaktierungselement 3 auf dem Biosensor 10 über eine oder mehrere Leiterbahnen 12 verbunden.

Es können sich weitere Funktionselemente, wie Arbeits-, und/oder Gegen-, und/oder Referenzelektroden und/oder Kontaktierungselemente 3 sowie Leiterbahnen 12 auf einer oder zwei weiteren der drei Außenoberflächen 9a, 9b, 9c befinden. Weiterhin können sich auch zusätzlich oder alternativ ein oder mehrere Funktionselemente wie Arbeitselektroden 5a, Gegenelektroden 5b, Referenzelektroden 5c oder Kontaktierungselemente 3 auf den Innenflächen 11 des Biosensors 10 befinden, was hier jedoch nicht gezeigt ist.

Wie bereits oben beschrieben, sind weitere dreidimensionale Ausgestaltungen des Biosensors 1 durch mehrfaches Knicken an mehreren Knickstellen 4 denkbar. So müssen die Knickstellen 4 nicht, wie in diesem Beispiel gezeigt, alle parallel zueinander verlaufen sondem können auch schräg oder quer zueinander verlaufen und können sich dabei auch kreuzen. Es können beispielsweise durch einen oder mehrere Knickvorgänge Bereiche mit Funktionselementen auf einer ersten Außenoberfläche 9a, von Bereichen ohne Funktionselemente auf einer zweiten Außenoberflächen 9b, getrennt werden. Des weiteren können ein Knickvorgang dazu dienen, den Biosensor 10 um eine Lanzette 6, beispielsweise eine Nadel, zu knicken, beispielsweise um diesen für eine Insertion an die Nadel zu fixieren.

### Bezugszeichenliste

- 1: Biosensor
- 2: Substrat
- 3: Kontaktierungselement
- 4: Knickstelle
- 5: Elektrode
- 5a: Arbeitselektrode
- 5b: Gegenelektrode
- 5c: Referenzelektrode
- 5d: Elektrodenbereich
- 6: Lanzette
- 7: Lanzettenspitze
- 8: Aussparung in Lanzettenspitze
- 9: Oberfläche des Substrats
- 9a: erste Außenoberfläche des Substrats
- 9b: zweite Außenoberfläche des Substrats
- 9c: dritte Außenoberfläche des Substrats
- 10: geknickter Biosensor
- 11: Innenfläche des Substrats
- 12: Leiterbahn
- 13: Substratzuschnitt
- 14: Einkerbung
- 15: Perforation
- 16: Furche
- 17: Falz
- 18: Verbreiterung
- 19: Kontaktierungsbereich
- 20: Lasche, Schaft
- 21: Längserstreckungsachse
- 22: Insertionskit
- 24: Stechelement
- 25: Substratteil

## Patentansprüche

1. Biosensor (1, 10) zum Insertieren in ein subcutanes Gewebe eines Benutzers, wobei der Biosensor eingerichtet ist zum qualitativen und/oder quantitativen Nachweis mindestens eines Analyten in einer Körperflüssigkeit, wobei der Biosensor (1, 10) mindestens ein flexibles Substrat (2) und mindestens eine Elektrode (5) auf mindestens einer Oberfläche (9) des Substrats (2) und mindestens ein Kontaktierungselement (3) aufweist, wobei das Kontaktierungselement (3) mit der Elektrode (5) verbunden ist, **dadurch gekennzeichnet, dass** das Substrat (2) mindestens eine Knickstelle (4) aufweist, an der das Substrat (2) mindestens zu einem Teil geknickt ist, so dass die Oberfläche (9) in zumindest zwei miteinander verbundene Außenoberflächen (9a, 9b, 9c) unterteilt ist.

2. Biosensor (1, 10) nach dem vorherigen Anspruch, **dadurch gekennzeichnet, dass** auf mindestens zwei der Außenoberflächen (9a, 9b, 9c) jeweils mindestens eine Elektrode (5) angeordnet ist.

3. Biosensor (1, 10) nach einem der vorhergehenden Ansprüche, **dadurch gekennzeichnet, dass** die Außenoberflächen (9a, 9b, 9c) mindestens zwei zueinander im Wesentlichen parallel angeordnete Außenoberflächen (9a, 9b, 9c) aufweisen.

4. Biosensor (1, 10) nach einem der vorherigen Ansprüche, **dadurch gekennzeichnet, dass** durch die Knickstelle (4) das Substrat (2) in mindestens zwei miteinander verbundene Substratteile (25) unterteilt wird, wobei die Substratteile (25) mindestens zu einem Teil miteinander verbunden sind, insbesondere verklebt sind.

5. Biosensor (1, 10) nach einem der vorherigen Ansprüche, **dadurch gekennzeichnet, dass** die mindestens eine Elektrode (5) mindestens eine Arbeitselektrode (5a) und/oder mindestens eine Referenzelektrode (5c) und/oder mindestens eine Gegenelektrode (5b) umfasst.

6. Biosensor (1, 10) nach einem der vorherigen Ansprüche, **dadurch gekennzeichnet, dass** sowohl auf der ersten als auch auf der zweiten Außenoberfläche (9a, 9b, 9c) mindestens ein Kontaktierungselement (3) und/oder mindestens eine Elektrode (5) angeordnet sind.

7. Biosensor (1, 10) nach einem der vorherigen Ansprüche, **dadurch gekennzeichnet, dass** die Knickstelle (4) in dem Kontaktierungselement (3) angeordnet ist.

8. Biosensor (1, 10) nach einem der vorherigen Ansprüche, **dadurch gekennzeichnet, dass** zwischen dem Kontaktierungselement (3) und der Elektrode (5) mindestens eine Leiterbahn (12) angeordnet ist.

9. Biosensor (1, 10) nach einem der vorherigen Ansprüche, **dadurch gekennzeichnet, dass** das Substrat (2) eine längliche Struktur aufweist und dass sich die eine Elektrode (5) und das Kontaktierungselement (3) an einander gegenüberliegenden Enden mindestens einer der Außenoberflächen (9a, 9b, 9c) befinden.

10. Substratzuschnitt (13) zur Herstellung eines Biosensors (1, 10) nach einem der vorhergehenden Ansprüche, wobei der Substratzuschnitt (13) mindestens ein flexibles Substrat (2) aufweist und mindestens eine Elektrode (5) auf mindestens einer Oberfläche (9) des Substrats (2) und mindestens ein Kontaktierungselement (3) aufweist, wobei das Kontaktierungselement (3) mit der Elektrode (5) verbunden ist, **dadurch gekennzeichnet, dass** das Substrat (2) mindestens eine Knickstelle (4) aufweist, an der das Substrat (2) mindestens zu einem Teil knickbar ist, so dass die Oberfläche (9) in zumindest zwei miteinander verbundene Außenoberflächen (9a, 9b, 9c) unterteilt wird, wobei die Knickstelle (4) mindestens eine Schwächung eines Substratmaterials des Substrats (2) umfasst.

11. Substratzuschnitt (13) nach dem vorherigen Anspruch, wobei die Knickstelle (4) in einer Weise ausgeformt ist ausgewählt aus: einem Falz (17), einer Perforation (15), einer Knicklinie mit einer Furche (16) oder Kombinationen hieraus.

12. Insertionskit (22) zum Insertieren eines Biosensors (1, 10) in ein subcutanes Gewebe eines Benutzers, umfassend mindestens einen Biosensor (1, 10) nach einem der vorhergehenden, einen Biosensor (1, 10) betreffenden Ansprüche, weiterhin umfassend ein Stechelement (24), wobei das Stechelement (24) insbesondere ausgewählt ist aus: einer Nadel, einer Lanzette (6), einer Flachlanzette, einer Rundlanzette oder einer Kanüle.

13. Insertionskit (22) nach dem vorhergehenden Anspruch, wobei das Stechelement (24) zwischen den beiden Außenoberflächen (9a, 9b, 9c) angeordnet ist.

14. Verfahren zur Herstellung eines Biosensors (1, 10) zum qualitativen und/oder quantitativen Nachweis mindestens eines Analyten in einer Körperflüssigkeit, insbesondere eines Biosensors (1, 10) nach einem der vorhergehenden, einen Biosensor (1, 10) betreffenden Ansprüche, mit den Schritten:
- Bereitstellen mindestens eines flexiblen Substrats (2),
- Aufbringen mindestens eines Kontaktierungselementes (3) und mindestens einer mit dem Kontaktierungselement (3) verbundenen Elektrode (5) auf mindestens eine Oberfläche (9) des Substrats (2),
**dadurch gekennzeichnet, dass** das Verfahren weiterhin folgenden Schritt umfasst:
- Knicken des Substrats (2) an mindestens einer Knickstelle (4), so dass die Oberfläche (9) in zumindest zwei miteinander verbundene Außenoberflächen (9a, 9b, 9c) unterteilt wird.

15. Verfahren nach dem vorhergehenden Anspruch, wobei der Biosensor (1) eine längliche Struktur aufweist und das Knicken in einer Weise erfolgt, ausgewählt aus: einem Knicken parallel zu einer Längserstreckungsachse (21) des Biosensors (1, 10); einem Knicken quer zu einer Längserstreckungsachse (21) des Biosensors (1, 10).

## Claims

1. A biosensor (1, 10) for insertion into subcutaneous tissue of a user, wherein the biosensor is set up for qualitative and/or quantitative detection of at least one analyte in a bodily fluid, wherein the biosensor (1, 10) comprises at least one flexible substrate (2) and at least one electrode (5) on at least one surface (9) of the substrate (2) and at least one contacting element (3), wherein the contacting element (3) is connected to the electrode (5), **characterized in that** the substrate (2) has at least one kink (4), at which the substrate (2) is at least partly kinked such that the surface (9) is subdivided into at least two interconnected outer surfaces (9a, 9b, 9c).

2. The biosensor (1, 10) as claimed in the preceding claim, **characterized in that** at least one electrode (5) is arranged in each case on at least two of the outer surfaces (9a, 9b, 9c).

3. The biosensor (1, 10) as claimed in one of the preceding claims, **characterized in that** the outer surfaces (9a, 9b, 9c) have at least two outer surfaces (9a, 9b, 9c) which are arranged substantially parallel to one another.

4. The biosensor (1, 10) as claimed in one of the preceding claims, **characterized in that** the substrate (2) is subdivided into at least two interconnected substrate pieces (25) by the kink (4), wherein the substrate pieces (25) are connected, more particularly adhesively bonded, to one another at least in part.

5. The biosensor (1, 10) as claimed in one of the preceding claims, **characterized in that** the at least one electrode (5) comprises at least one working electrode (5a) and/or at least one reference electrode (5c) and/or at least one counter electrode (5b).

6. The biosensor (1, 10) as claimed in one of the preceding claims, **characterized in that** at least one contacting element (3) and/or at least one electrode (5) are arranged on both the first and the second outer surface (9a, 9b, 9c).

7. The biosensor (1, 10) as claimed in one of the preceding claims, **characterized in that** the kink (4) is arranged in the contacting element (3).

8. The biosensor (1, 10) as claimed in one of the preceding claims, **characterized in that** at least one conductor track (12) is arranged between the contacting element (3) and the electrode (5).

9. The biosensor (1, 10) as claimed in one of the preceding claims, **characterized in that** the substrate (2) has an elongate structure and **in that** the one electrode (5) and the contacting element (3) are situated at mutually opposite ends of at least one of the outer surfaces (9a, 9b, 9c).

10. A substrate blank (13) for producing a biosensor (1, 10) as claimed in one of the preceding claims, wherein the substrate blank (13) has at least one flexible substrate (2) and at least one electrode (5) on at least one surface (9) of the substrate (2) and at least one contacting element (3), wherein the contacting element (3) is connected to the electrode (5), **characterized in that** the substrate (2) has at least one kink (4) at which the substrate (2) can be kinked at least in part such that the surface (9) is subdivided into at least two interconnected outer surfaces (9a, 9b, 9c), wherein the kink (4) comprises at least one weakening of a substrate material of the substrate (2).

11. The substrate blank (13) as claimed in the preceding claim, wherein the kink (4) is formed in a fashion selected from the following: a slit (17), a perforation (15), a kink line with a groove (16) or a combination therefrom.

12. An insertion kit (22) for inserting a biosensor (1, 10) into subcutaneous tissue of a user, comprising at least one biosensor (1, 10) as claimed in one of the preceding claims relating to a biosensor (1, 10), furthermore comprising a piercing element (24), wherein the piercing element (24) is selected in particular from: a needle, a lancet (6), a flat lancet, a round lancet or a cannula.

13. The insertion kit (22) as claimed in the preceding claim, wherein the piercing element (24) is arranged between the two outer surfaces (9a, 9b, 9c).

14. A method for producing a biosensor (1, 10) for the qualitative and/or quantitative detection of at least one analyte in a bodily fluid, more particularly a biosensor (1, 10) as claimed in one of the preceding claims relating to a biosensor (1, 10), comprising the following steps:
- providing at least one flexible substrate (2),
- applying at least one contacting element (3) and at least one electrode (5) connected to the contacting element (3) to at least one surface (9) of the substrate (2),
**characterized in that** the method furthermore comprises the following step:
- kinking the substrate (2) at at least one kink (4) such that the surface (9) is subdivided into at least two interconnected outer surfaces (9a, 9b, 9c).

15. The method as claimed in the preceding claim, wherein the biosensor (1) has an elongate structure and the kinking takes place in a manner selected from: kinking parallel to an axis of longitudinal extent (21) of the biosensor (1, 10); kinking at an angle to an axis of longitudinal extent (21) of the biosensor (1, 10).

## Revendications

1. Biocapteur (1, 10) destiné à être introduit dans un tissu sous-cutané d'un utilisateur, ledit biocapteur étant adapté pour la justification qualitative et/ou quantitative d'au moins un analyte dans un liquide corporel, le biocapteur (1, 10) comportant au moins un substrat (2) flexible et au moins une électrode (5) située sur au moins une surface (9) du substrat (2) et au moins un élément de mise en contact (3), l'élément de mise en contact (3) étant relié à l'électrode (5), **caractérisé en ce que** le substrat (2) comporte au moins une pliure (4) où le substrat (2) est plié au moins en partie de façon à diviser la surface (9) en au moins deux surfaces extérieures (9a, 9b, 9c) reliées l'une à l'autre.

2. Biocapteur (1, 10) selon la revendication précédente, **caractérisé en ce que** sur au moins deux des surfaces extérieures (9a, 9b, 9c) est disposée à chaque fois au moins une électrode (5).

3. Biocapteur (1, 10) selon l'une des revendications précédentes, **caractérisé en ce que** les surfaces extérieures (9a, 9b, 9c) comportent au moins deux surfaces extérieures (9a, 9b, 9c) disposées de façon sensiblement parallèle l'une à l'autre.

4. Biocapteur (1, 10) selon l'une des revendications précédentes, **caractérisé en ce que** la pliure (4) divise le substrat (2) en au moins deux parties de substrat (25) reliées l'une à l'autre, les parties de substrat (25) étant reliées l'une à l'autre, en particulier collées ensemble, au moins en partie.

5. Biocapteur (1, 10) selon l'une des revendications précédentes, **caractérisé en ce que** la au moins une électrode (5) comprend au moins une électrode de travail (5a) et/ou au moins une électrode de référence (5c) et/ou au moins une contre-électrode (5b).

6. Biocapteur (1, 10) selon l'une des revendications précédentes, **caractérisé en ce que** sur la première et sur la deuxième surface extérieure (9a, 9b, 9c) sont disposés au moins un élément de mise en contact (3) et/ou au moins une électrode (5).

7. Biocapteur (1, 10) selon l'une des revendications précédentes, **caractérisé en ce que** la pliure (4) est agencée dans l'élément de mise en contact (3).

8. Biocapteur (1, 10) selon l'une des revendications précédentes, **caractérisé en ce que** entre l'élément de mise en contact (3) et l'électrode (5) est disposée au moins une piste conductrice (12).

9. Biocapteur (1, 10) selon l'une des revendications précédentes, **caractérisé en ce que** le substrat (2) a une structure allongée et **en ce que** l'électrode (5) et l'élément de mise en contact (3) sont situés aux extrémités opposées d'au moins une des surfaces extérieures (9a, 9b, 9c).

10. Ébauche de substrat (13) pour la fabrication d'un biocapteur (1, 10) selon l'une des revendications précédentes, l'ébauche de substrat (13) comportant au moins un substrat (2) flexible et au moins une électrode (5) située sur au moins une surface (9) du substrat (2) et au moins un élément de mise en contact (3), l'élément de mise en contact (3) étant relié à l'électrode (5), **caractérisé en ce que** le substrat (2) comporte au moins une pliure (4) où le substrat (2) peut être plié au moins en partie de façon à diviser la surface (9) en au moins deux surfaces extérieures (9a, 9b, 9c) reliées l'une à l'autre, la pliure (4) comprenant au moins un affaiblissement dans un matériau du substrat (2).

11. Ébauche de substrat (13) selon la revendication précédente, la pliure (4) étant formée selon une méthode choisie parmi : un pli (17), une perforation (15), une ligne de pliage avec une rainure (16) ou des combinaisons de ceux-ci.

12. Kit d'introduction (22) destiné à introduire un biocapteur (1, 10) dans un tissu sous-cutané d'un utilisateur, comprenant au moins un biocapteur (1, 10) selon l'une des revendications précédentes concernant un biocapteur (1, 10), comprenant en outre un élément d'incision (24), l'élément d'incision (24) étant choisi en particulier parmi : une aiguille, une lancette (6), une lancette plate, une lancette ronde ou une canule.

13. Kit d'introduction (22) selon la revendication précédente, l'élément d'incision (24) étant disposé entre les deux surfaces extérieures (9a, 9b, 9c).

14. Procédé de fabrication d'un biocapteur (1, 10) destiné à la justification qualitative et/ou quantitative d'au moins un analyte dans un liquide corporel, en particulier d'un biocapteur (1, 10) selon l'une des revendications précédentes concernant un biocapteur (1, 10), comprenant les étapes :
- approvisionner au moins un substrat (2) flexible,
- déposer au moins un élément de mise en contact (3) et au moins une électrode (5) reliée à l'élément de mise en contact (3) sur au moins une surface (9) du substrat (2),
**caractérisé en ce que** le procédé comprend en outre l'étape suivante :
- plier le substrat (2) au niveau d'au moins une pliure (4) de façon à diviser la surface (9) en au moins deux surfaces extérieures (9a, 9b, 9c) reliées l'une à l'autre.

15. Procédé selon la revendication précédente, le biocapteur (1) ayant une structure allongée et le pliage étant effectuée selon une méthode choisie parmi: un pliage parallèle à un axe d'extension longitudinale (21) du biocapteur (1, 10); un pliage transversal à un axe d'extension longitudinale (21) du biocapteur (1, 10).
